(19) Europäisches Patentamt
European Patent Office
Office européen des brevets

(11) EP 4 768 480 A1

(12) **EUROPEAN PATENT APPLICATION**
published in accordance with Art. 153(4) EPC

(43) Date of publication:
**01.07.2026 Bulletin 2026/27**

(21) Application number: **24842454.1**

(22) Date of filing: **23.07.2024**

(51) International Patent Classification (IPC):
***C07D 409/12*** (2006.01) ***A61K 31/4406*** (2006.01)
***A61K 31/5377*** (2006.01) ***A61K 31/505*** (2006.01)
***A61P 9/10*** (2006.01) ***A61P 1/16*** (2006.01)
***A61P 3/04*** (2006.01) ***A61P 3/10*** (2006.01)

(52) Cooperative Patent Classification (CPC):
**A61K 31/4406; A61K 31/4436; A61K 31/444; A61K 31/501; A61K 31/505; A61K 31/5377; A61P 1/00; A61P 1/16; A61P 3/04; A61P 3/10; A61P 5/50; A61P 7/10; A61P 9/10; A61P 11/00; A61P 25/28;** (Cont.)

(86) International application number:
**PCT/CN2024/106985**

(87) International publication number:
**WO 2025/016469 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Fudan University**
**Shanghai 200433 (CN)**

(72) Inventors:
- **ZHOU, Lu**
  **Shanghai 200433 (CN)**
- **HONG, Jiaxu**
  **Shanghai 200433 (CN)**
- **YANG, Jintong**
  **Shanghai 200433 (CN)**
- **WANG, Wei**
  **Shanghai 200433 (CN)**
- **ZHANG, Xinchen**
  **Shanghai 200433 (CN)**
- **YE, Deyong**
  **Shanghai 200433 (CN)**

(74) Representative: **Metida**
**Gyneju str. 16**
**01109 Vilnius (LT)**

# (54) ALKOXY THIOPHENE ACYL ARYLAMINE COMPOUNDS AND USE THEREOF

(57) The present disclosure provides alkoxy thiophene acyl arylamine compounds and a use thereof, comprising alkoxy thiophene acyl arylamine compounds having a structure as shown in formula (1), or pharmaceutically acceptable salts thereof. The alkoxy thiophene acyl arylamine compounds provided by the present disclosure are a class of novel sphingomyelin synthase inhibitors, which have significant inhibitory activity against SMS2, ideal physical and chemical properties such as stability and water solubility, and low potential for toxic side effects, and can be further made into a drug for preventing or treating diseases related to abnormal sphingomyelin levels



Processed by Luminess, 75001 PARIS (FR)

**(Cont. next page)**

(52) Cooperative Patent Classification (CPC): (Cont.)
**A61P 27/02; A61P 29/00; A61P 35/00;**
**C07D 409/12; C07D 409/14; C07F 9/6558**

## Description

## FIELD

The present disclosure belongs to the field of pharmaceutical chemistry, and more particularly relates to alkoxy thiophene acyl arylamine compounds and use thereof.

## BACKGROUND

Atherosclerosis (AS) is one of the main pathological bases of many cardiovascular and cerebrovascular diseases, and therefore, the research on anti-atherosclerotic drugs has become a hot spot in the field of current drug research and development.

Studies have proposed a variety of potential drug targets for anti-atherosclerosis, such as sphingomyelin synthase inhibitors, PPAR agonists, infusion carrier lipoproteins, liver X receptor activators, phospholipid transfer proteins (PLTP) inhibitors, and the like. Especially sphingomyelin (SM) and metabolic enzymes thereof to mediate a series of cellular processes while causing lipoproteins to change, indicating that they play an important role in the development of atherosclerosis. It is believed that reducing the level of sphingomyelin in plasma or inhibiting the synthesis of SM can achieve the purpose of slowing or preventing the development of atherosclerosis.

In addition, researchers found that the sphingomyelin synthase (SMS) can adjust ceramide and lecithin (PC) to synthesize SM, which is the key enzyme of the last step of the de novo synthesis pathway of sphingomyelin. The sphingomyelin synthase mainly has three subtypes, sphingomyelin 1 (SMS1), sphingomyelin 2 (SMS2), and sphingomyelin-related protein (SMSr). Studies show that inhibiting SMS2 reduction can increase sphingomyelin levels, which may provide a new method for treating atherosclerosis. SMS2 has potential as a novel target for atherosclerosis, and an SMS2-selective inhibitor will likely become a novel anti-atherosclerosis therapeutic drug. In addition, studies have found that SMS2 deficiency can prevent high-fat diet-induced obesity and insulin resistance, while in the liver of SMS2 gene knockout mice, it is difficult to observe large mature fat plaques, indicating that SMS2 participates in the formation of liver fat plaque and can induce the occurrence of obesity and type II diabetes (Mitsutake et al., 2011). Sphingomyelin small molecule inhibitors will likely be used to prevent and treat metabolic syndrome such as type II diabetes, obesity and fatty liver. One study reported that SMS2 gene knockout can significantly improve inflammation, insulin tolerance and other aspects of metabolic syndrome (Mitsutake et al., 2011) in mice fed with high fat. The SMS2 small molecule inhibitor will likely be used to prevent and treat inflammation-related diseases such as colitis, colon cancer, pulmonary edema, and lung injury. Studies have found that SMS2 can promote the EMT process of breast cancer cells (Zheng et al., 2019), colon cancer cell drug resistance (Jin et al., 2016), the growth and metastasis of ovarian cancer (Jing et al., 2021), macrophage M2 polarization (Deng et al., 2020) in breast cancer tumor microenvironment, infiltration of malignant lymphoma (Taniuchi et al., 2020), and the growth of leukemia cells (JP2021-165292A; WO2005032492), etc. Sphingomyelin small molecule inhibitors will likely be used to prevent and treat malignant tumors, such as triple-negative breast cancer, colon cancer, leukemia, ovarian cancer, etc. The deletion of SMS2 can inhibit the content and aggregation of sphingomyelin in the brain, reducing the content of Aβ (Kidani et al., 2012; Yupyama et al., 2014; Hsiao et al., 2013). SMS2 small molecule inhibitors will likely be used to prevent and treat central system diseases such as neurodegenerative diseases such as Alzheimer's disease. Literatures disclosed some lipid abnormalities in the tear fluid of dry eye, including sphingomyelin (Ham et al., 2006; Paranjpe et al. 2019), which can be used to prevent and alleviate sphingomyelin abnormal metabolismrelated diseases such as dry eye syndrome.

With the depth of SMS biological function research, it was found that SMS2 is associated with the occurrence and development of various diseases In recent years, various SMS inhibitors have been found successively and in part have been applied to certain disease treatment model evaluations. Aimin Meng et al. treated U937 human monocyte leukemia cells with a selective tumor cytotoxic agent **D609**, and found that the cell death induced by **D609** is time and concentration-dependent. This process was accompanied by a high level of ceramide and a low level of sphingomyelin (SM) and diacylglycerol (DAG) in the cells. When cells were treated with **D609** in combination with either a PKC inhibitor or agonist, it was observed that the PKC inhibitor enhanced apoptosis, while the agonist reversed this effect, thereby providing evidence that SMS is a potential target of **D609** (Meng et al., 2004). SalmaY et al. found that **Jaspine B,** isolated from sponge, significantly inhibits the SMS activity of melanoma cells and induces melanoma cell apoptosis (Sala et al., 2009). Mahala M. M. Swamy et al. synthesized series of ceramide analogs, including compound **17j**, which exhibit certain SMS inhibitory effects (Swamy et al., 2018). Muhmad Aqmal Othman et al. found that **Malabarie C,** a compound isolated from Myrichtca Cinema King fruit, inhibits SMS2 but has a low selectivity for SMS2 and SMS1. It significantly reduces the weight gain in HFD-induced mice and improves glucose tolerance and liver steatosis (Swamy et al., 2018). Ryutaro Adachi et al. established a radioactive hydrogen substrate-labeled SMS detection system, and obtained a class of quinolone compounds (such as compound **3**) with better SMS2 inhibitory activity and selectivity by high-throughput screening and structural optimization (Adachi et al., 2017). Similarly, Takafumi Yukawa et al. modified the quinolone compounds

described above to obtain a class of 1,8-diazonaphthalen-2-one compounds, such as compound **37** (Yukawa et al., 2020). Xiaodong Deng et al. screened and designed a class of 2-benzyloxyphenylacetonitrile derivatives by virtual screening and enzymatic activity assays, with a preliminary SM2 inhibitory activity, such as **D2** (Deng et al., 2014). Ya-Li et al. obtained a class of SAPA series compounds with good inhibitory activity against SMS1 by means of virtual screening, such as **SAPA 1a** (Li et al., 2015). Xiang-Yu Qi et al. synthesized a class of benzoxazole derivatives with better SMS2 inhibitory activity and selectivity by skeleton transition binding molecule docking, such as **QY16**. Mingguang Mo, etc. synthesized a class of benzoisoxazole derivatives through skeleton transition and ring closure, and found that **15 w** of such compounds have good inhibitory activity and selectivity with good pharmacokinetic properties of SMS2, can significantly inhibit the level of inflammatory factors in db/db mice at an oral dose of 50 mg/kg, reduce LDL-C and increase HDL-C levels (Moetal.2018). Yali Li et al. designed and obtained a class of salicylamide derivatives by means of electronic and other means, and found that **Ly93** in this class of compounds has a higher SMS2 inhibitory activity and selectivity, can inhibit the activity of liver SMS in a dose-dependent manner, reduce the level of plasma SM, and finally inhibit the development of atherosclerosis. In vitro test results show that **Ly93** reduces apoB secretion of Huh7 cells in a dose-dependent manner, reduces the SMS activity of macrophages, and increases the outflow of cholesterol; meanwhile, **Ly93** can inhibit LPS-mediated macrophage pro-inflammatory factors and chemokine secretion (Li et al.,2019). Hadya Virupaksha Deepak et al. found that **Daurichromonic acid** (DCA), a compound isolated from Rhododendron daurium plants, inhibits SMS activity and exhibits a preliminary activity of inhibiting the aggregation of Aβ ($EC_{50}$ = 57 μM) (Deepak et al., 2020). In general, although many SMS inhibitors have been found to be found in succession, the type of structure of the SMS2 inhibitor and the formation need to be further optimized.

## SUMMARY

A first object of the present disclosure is to provide an alkoxy thiophene acyl arylamine compound (including a prodrug which is a carboxyl/phosphate compound derived from the alkoxy thiophene acyl arylamine compound) as a sphingomyelin synthase inhibitor having a significant physical and chemical property such as significant inhibitory activity for SMS2, stability, and water solubility.

A second object of the present disclosure is to provide a pharmaceutical composition containing an alkoxy thiophene acyl arylamine compound (including a prodrug which is a carboxyl/phosphate compound derived from the alkoxy thiophene acyl arylamine compound) or pharmaceutically acceptable salts thereof.

A third object of the present disclosure is to provide a pharmaceutical composition containing an alkoxy thiophene acyl arylamine compound (including a prodrug which is a carboxyl/phosphate compound derived from the alkoxy thiophene acyl arylamine compound) or pharmaceutically acceptable salts thereof for use in the preparation of medicaments.

To achieve the above objects, the present disclosure provides an alkoxy thiophene acyl arylamine compound of Formula (I), or a pharmaceutically acceptable salt thereof,

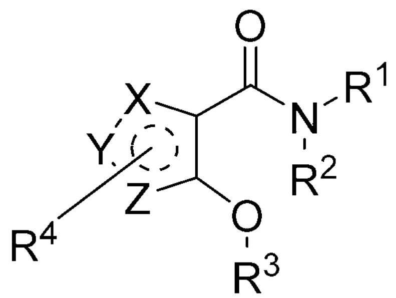

(I)

wherein X, Y and Z are each independently selected from the group consisting of carbon atom and sulfur atom;
$R^1$ is phenyl, pyridyl, pyridazinyl, pyrimidinyl or thiazolyl, wherein such groups are unsubstituted or substituted with 1, 2, 3, 4 or 5 substituents independently selected from the group consisting of halogen, methyl, hydroxyl, methoxy, trifluoromethyl, trifluoromethoxy, cyano, and nitro;
$R^2$ is hydrogen, an alkyl group having 1 to 3 carbon atoms, or cycloalkyl;
$R^3$ is a substituted or unsubstituted hydrocarbyl group;
$R^4$ is one or two groups selected from the group consisting of hydrogen, alkyl group having 1 to 3 carbon atoms, halogen, and nitro.

As a preferred solution, X and Y are each independently selected from the group consisting of carbon atom and sulfur atom, and Z is carbon atom.

As a preferred solution, $R^3$ is a group having one of the following structures:

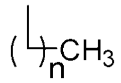 , 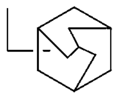 , 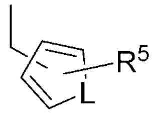 , 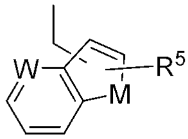 , 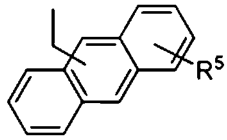 ,

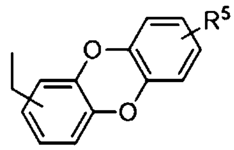 .

wherein n is an integer from 1 to 10;
W is carbon atom or nitrogen atom;
L, M are each independently selected from the group consisting of -C=C-, sulfur atom and oxygen atom;
$R^5$ is 1 to 5 groups selected from the group consisting of hydrogen, a substituted or unsubstituted alkyl group having 1 to 4 carbon atoms, a substituted or unsubstituted alkoxy group having 1 to 20 carbon atoms, halogen, acyl group, nitro, cyano, hydroxyl, carboxylate group, phosphate group and heteroaromatic ring.

As a preferred solution, the substituted or unsubstituted alkoxy group has 1 to 15 carbon atoms.

As a preferred solution, the alkoxy thiophene acyl arylamine compound is:

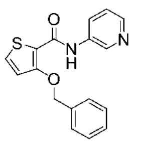
1-1

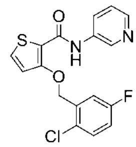
1-2

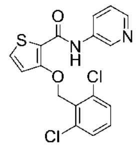
1-3

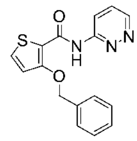
1-4

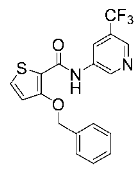
1-5

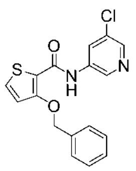
1-6

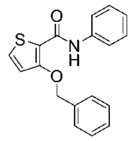
1-7

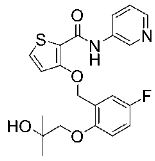
1-8

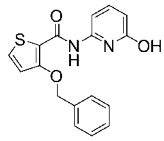
1-9

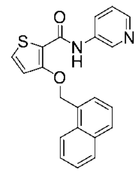
1-10

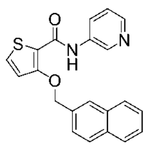
1-11

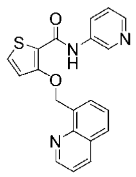
1-12

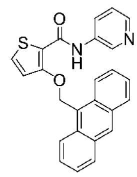
1-13

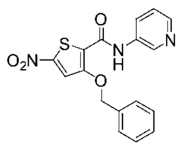
1-14

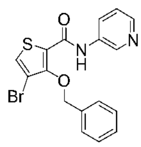
1-15

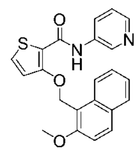
1-16

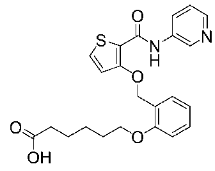
1-17

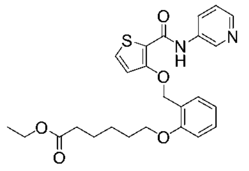
1-18

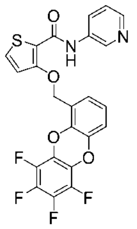
1-19

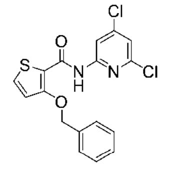
1-20

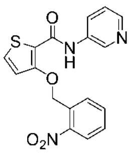
1-21

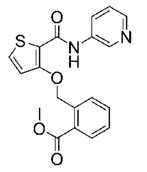
1-22

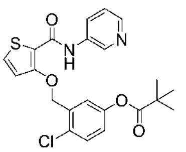
1-23

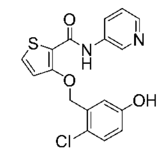
1-24

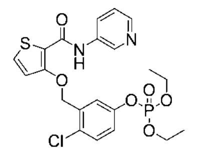
1-25

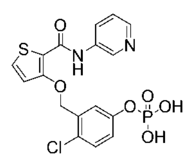
1-26

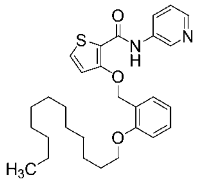
1-27

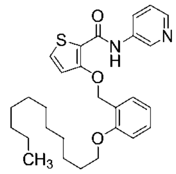
1-28

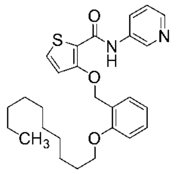
1-29

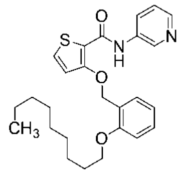
1-30

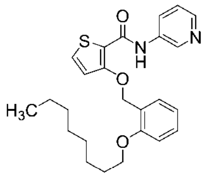
1-31

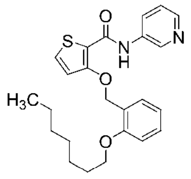
1-32

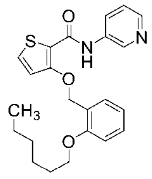
1-33

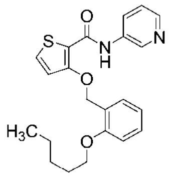
1-34

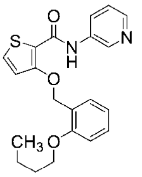
1-35

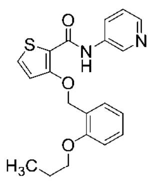
1-36

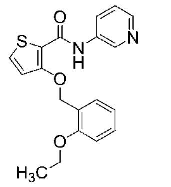
1-37

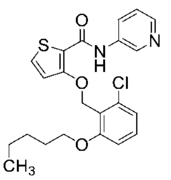
1-38

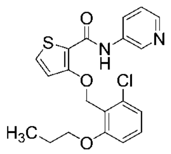
1-39

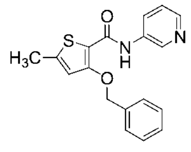
1-40

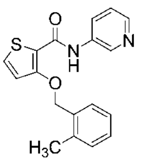
1-41

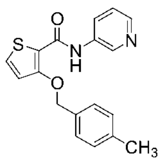
1-42

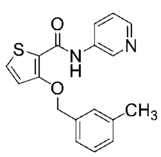
1-43

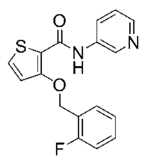
1-44

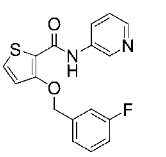
1-45

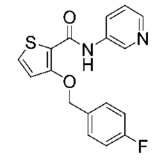
1-46

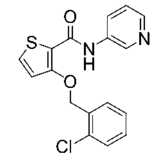
1-47

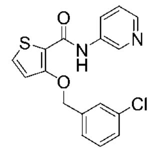
1-48

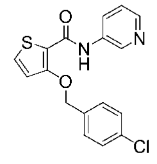
1-49

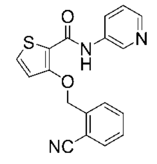
1-50

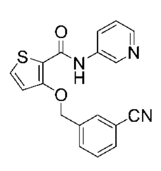
1-51

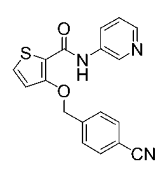
1-52

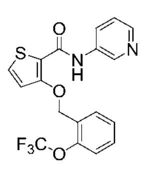
1-53

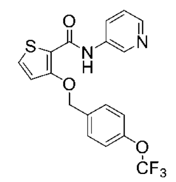
1-54

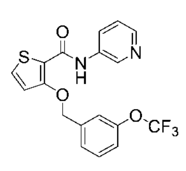
1-55

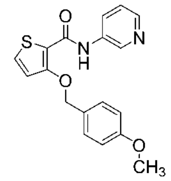
1-56

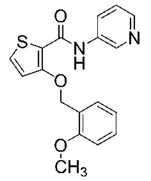
1-57

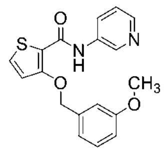
1-58

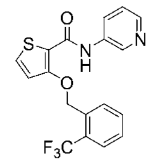
1-59

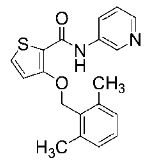
1-60

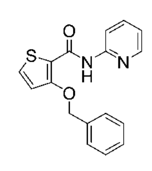
1-61

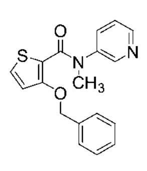
1-62

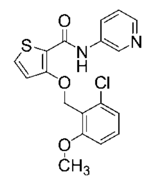
1-63

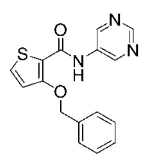
1-64

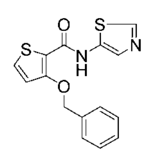
1-65

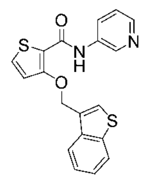
1-66

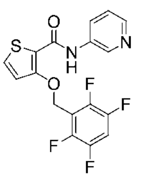
1-67

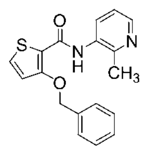
1-68

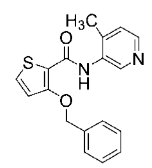
1-69

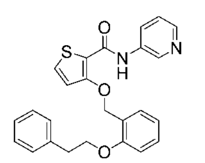
1-70

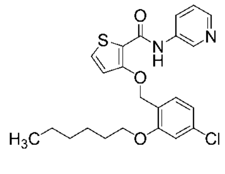
1-71

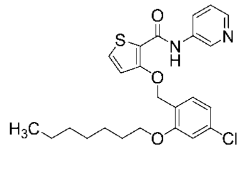
1-72

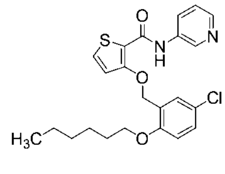
1-73

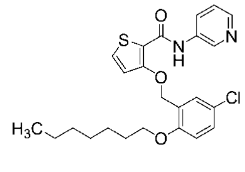
1-74

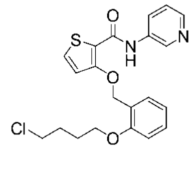
1-75

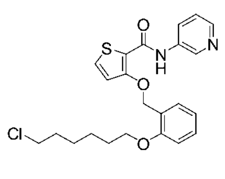 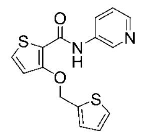 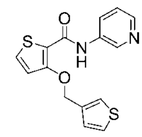 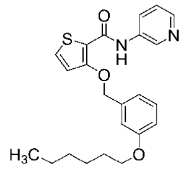 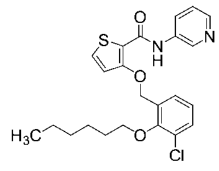

1-76 1-77 1-78 1-79 1-80

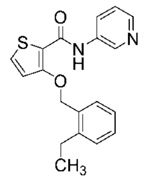 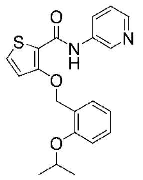 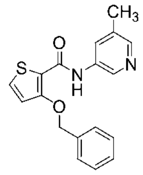 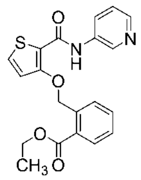 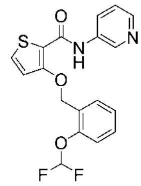

1-81 1-82 1-83 1-84 1-85

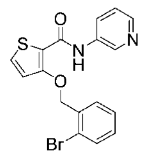 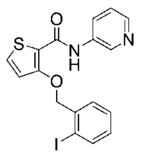 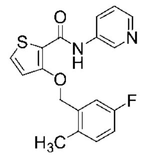 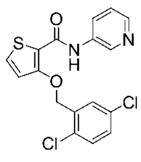 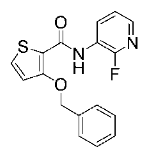

1-86 1-87 1-88 1-89 1-90

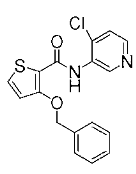 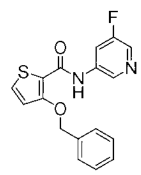 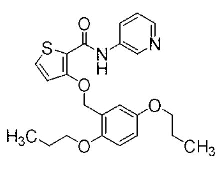 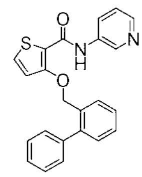 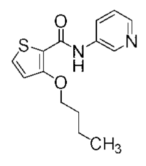

1-91 1-92 1-93 1-94 1-95

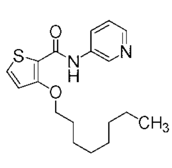 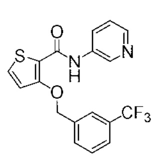 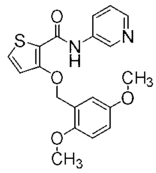 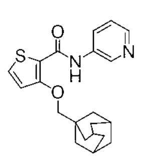 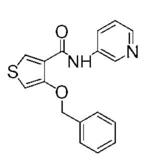

1-96 1-97 1-98 1-99 1-100 .

As a preferred solution, the pharmaceutically acceptable salt is a hydrochloride salt, a hydrobromide salt, a tartrate salt, a mesylate salt, a sodium salt, a potassium salt, a calcium salt, an ammonium salt, or an amino acid salt.

The compound of the present disclosure contains an alkaline group and an acid salt, and a salt of a derivative can be formed by ordinary means, including an organic acid salt such as acetate, citrate, fumarate, maleate, oxalate, malate, citrate, succinate, tartrate, lactate, camphorsulfonate, benzenesulfonate, p-toluenesulfonate, methanesulfonate, trifluoroacetate, triflate, and the like; Inorganic acid salts such as hydrohaloacids (hydrofluoric acid, hydrochloric acid, hydrobromic acid, hydroiodic acid) salts, sulfates, phosphates, nitrates, and the like. or with amino acids, such as glutamic acid or aspartic acid, which can form glutamate or aspartic acid salts, and the preferred salts are hydrochloride and hydrobromide; similarly, in the present disclosure, some of the compounds contain acidic groups which can be salts with bases, and salts of derivatives can be formed by ordinary means, including sodium salts, potassium salts, calcium salts, ammonium salts or salts with amino acids such as lysine, arginine, histidine and the like, and the preferred salts are sodium salts and potassium salts.

To achieve the second object of the present disclosure, the present disclosure provides a pharmaceutical

composition comprising a therapeutically effective amount of at least one of the alkoxy thiophene acyl arylamine compound of Formula (I) or a pharmaceutically acceptable salt thereof.

The pharmaceutical composition of the present disclosure may also comprise one or more pharmaceutically acceptable carriers including conventional diluents, excipients, fillers, binders, wetting agents, disintegrants, absorption promoters, surfactants, adsorption carriers, lubricants, etc. in the pharmaceutical field. If necessary, the pharmaceutical composition may also comprise flavoring agents, sweeteners, etc.

The pharmaceutical composition of the present disclosure can be prepared in any form, such as granules, powders, tablets, coated tablets, capsules, pills, syrups, drops, solutions, suspensions and emulsions, or sustained release formulations of active ingredients, wherein the capsules include hard or soft gelatin capsules, granules and powders can be in the form of noneffervescent or effervescent forms.

The pharmaceutical composition of the present disclosure can be administered by various routes according to conventional methods, including oral, intravenous, intra-arterial, intraperitoneal, intrathoracic, transdermal, nasal, inhalation, rectal, ocular, and subcutaneous introduction.

To achieve the third object of the present disclosure, the present disclosure provides the use of the alkoxy thiophene acyl arylamine compound or a pharmaceutically acceptable salt thereof in the preparation of a small-molecule inhibitor of sphingomyelin synthase.

In the present disclosure, the inhibitory activity of the alkoxy thiophene acyl arylamine compound of Formula (I) against the sphingomyelin synathase is determined through the high-performance liquid chromatography (HPLC) fluorescence quantitative detection method reported in a literature (Deng et al. 2012), and the activity change of the inhibitor in the conversion of ceramide to sphingomyelin catalyzed sphingomyelin synathase was calculated by means of the change of the content of NBD-ceramide and NBD-spherelin therein. Based on the activity test experiments of the high-performance liquid chromatography (HPLC) fluorescence quantification, it shows that the alkoxy thiophene acyl arylamine compounds of formula (1), including a prodrug which is a carboxyl/phosphate compound derived from the alkoxy thiophene acyl arylamine compound, has a micro/sub-micromole SMS2 inhibitory activity, and the inhibitory activity of the compound on SMS2 is determined through the high-performance liquid chromatography (HPLC) fluorescence quantitative method.

The present disclosure also provides the alkoxy thiophene acyl arylamine compound, or a pharmaceutically acceptable salt thereof, for use in the preparation of medicaments for preventing or treating diseases caused by abnormal increase in sphingomyelin levels.

As a preferred solution, the diseases caused by abnormal increase in sphingomyelin levels include atherosclerosis, obesity, insulin resistance, fatty liver disease, type II diabetes, enteritis, pulmonary edema, lung injury, malignant tumor, alzheimer's disease, and dry eye syndrome. The present disclosure further provides evaluation data of the therapeutic effect of a pharmaceutical composition comprising the alkoxy thiophene acyl arylamine compound, or a pharmaceutically acceptable salt thereof, on dry eye mouse model.

Through the experiments in the present disclosure, it is demonstrated that the compound disclosed in the present disclosure has a significant inhibitory activity on SMS2, and ideal physicochemical properties such as stability and water solubility. Due to the absence of potential toxic groups, the compound disclosed in the present disclosure exhibits minimal potential toxic side effects, making it a suitable medicaments for preventing or treating diseases caused by abnormal increase in sphingomyelin levels, including metabolic syndrome related to metabolic disorders, such as atherosclerosis, obesity, insulin resistance, fatty liver and type II diabetes; diseases related to inflammation, such as enteritis, pulmonary edema, and lung injury; malignant tumors, such as triple-negative breast cancer, colon cancer, leukemia, ovarian cancer, etc.; central system diseases, such as neurodegenerative diseases such as Alzheimer's disease or dry eye syndrome, and the like.

The term "halogen" refers to fluorine, chlorine, bromine and iodine, and preferably fluorine and chlorine.

The term "alkyl group" comprises saturated aliphatic groups, including linear alkyl groups (such as methyl, ethyl, propyl, butyl, pentyl, hexyl, heptyl, octyl, nonyl, decyl, etc.), branched alkyl groups (such as isopropyl, tert-butyl, isobutyl, etc.), cycloalkyl groups (such as cyclopropyl, cyclopentyl, cyclohexyl, cycloheptyl, and cyclooctyl), alkyl-substituted cycloalkyl groups, and cycloalkyl-substituted alkyl groups. In certain embodiments, the linear or branched alkyl group has 4 or less carbon atoms on its chain.

The term "alkoxy group" comprises substituted and unsubstituted alkyl groups covalently linked to an oxygen atom. In certain embodiments, the linear or branched alkoxy has 20 or fewer carbon atoms on its chain, more preferably 15 or less carbon atoms.

The advantages of the present disclosure are that the alkoxythiophene carboxamide compound (including the carboxyl/phosphate compound derived therefrom) provided by the present disclosure is a novel sphingomyelin synthase inhibitor with a novel structure, which has a significant physical and chemical properties such as significant inhibitory activity, stability and water solubility for SMS2, and does not contain a potential toxic group, and the potential toxic side effect is small, and can further be made as a drug for preventing or treating related diseases with abnormal sphingomyelin levels.

The alkoxy thiophene acyl arylamine compound, including a prodrug which is a carboxyl/phosphate compound derived from the alkoxy thiophene acyl arylamine, provided in the present disclosure is a novel sphingomyelin synathase inhibitor with a novel structure, which has a significant inhibitory activity on SMS2, ideal physicochemical properties such as stability and water solubility, and minimal potential toxic side effects due to the absence of potential toxic groups, and can further be made as medicaments for preventing or treating diseases caused by abnormal increase in sphingomyelin levels.

## BRIEF DESCRIPTION OF THE DRAWINGS

Figure 1. shows the sodium fluorescein staining scores of mouse eyes, wherein the pathological groups (including carrier group, cyclosporine group, group of the compound **1-2**, and group of the compound **1-31**) exhibited significantly higher staining scores compared to normal mice (physiological control group). Furthermore, both SMS2 inhibitors and cyclosporine could reduce the dry eye pathological scores in the former. *p<0.05, **p<0.01, ****p<0.0001.

Figure 2. shows the photograph of the sodium fluorescein staining of mouse eyes, wherein the pathological groups (including carrier group, cyclosporine group, group of the compound **1-2**, and group of the compound **1-31**) show an increase in the green area compared to normal mice (physiological control group) after staining, indicating an increase in the degree of corneal damage. Compared to the carrier group, both SMS2 inhibitor and cyclosporine can reduce the area of corneal damage, with the former showing a more significant effect.

Figure 3 shows the statistics of tear secretion of mice, wherein compared to normal mice (physiological control group), the cotton thread wetting length was reduced in the pathological groups (including vehicle, cyclosporine, **1-2**, and **1-31** groups), indicating a decrease in tear secretion. Compared to the vehicle group, both SMS2 inhibitor and cyclosporine can promote tear secretion in mice, with the former being more significant, *p<0.05, ****p<0.0001. r being more significant, *p<0.05, ****p<0.0001.

## DETAILED DESCRIPTION

The present disclosure will be described in detail below with reference to specific embodiments. One skilled in the relevant art will understand that the embodiments disclosed hereinafter are to provide a thorough understanding of the present disclosure, rather than limiting the present disclosure.

### Example 1

### Preparation of 3-benzyloxy-N-(pyridin-3-yl)thiophene-2-carboxamide of formula 1-1

### 1) Synthesis of 3-(benzyloxy)thiophene-2-carboxylate

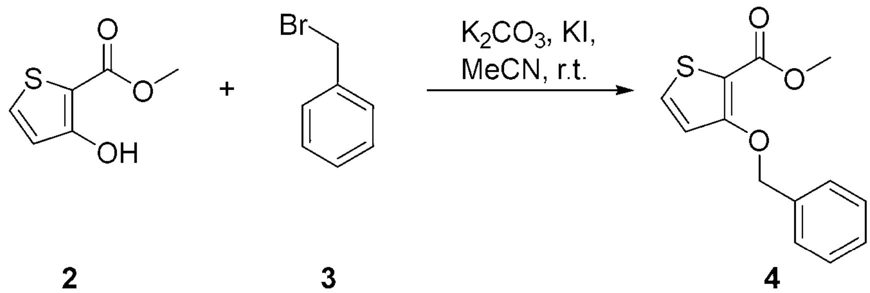


2 3 4

5 g (0.03 mol, 1.0 eq) of compound 2, 13 g of potassium carbonate (0.09 mol, 3.0 eq), 500 mg of potassium iodide were weighed and placed into a 250 mL round bottom flask, and 100 mL of acetonitrile were added thereto for dissolution. After 8.1 g (0.045 mol, 1.5 eq) of compound 3 were added while stirring, the reaction solution was stirred overnight at room temperature until the reaction was determined to be complete by TLC. The reaction solution was then filtered and evaporated to obtain 7.4 g of yellow oil, with a yield of 94%, and MS (ESI) (m/z): 249[M+H]+. The product can be used for the next reaction without further purification.

**2) Synthesis of 3-(benzyloxy)thiophene-2-carboxylic acid**

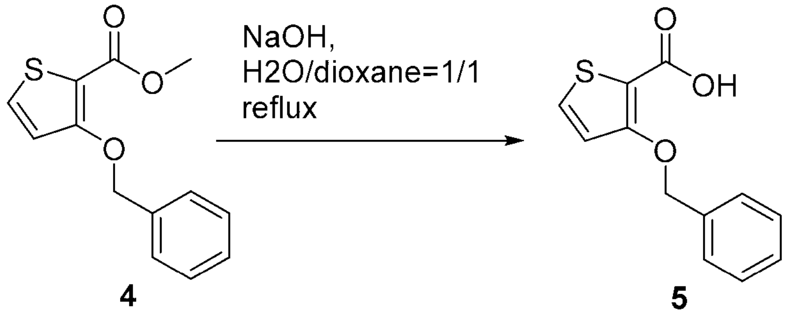


**4** **5**

7 g (0.03mol, 1.0eq) of compound 4 were weighed and placed into a 250 mL round bottom flask, and 100 mL of hydroxide solution(2mol/L, $H_2O$/dioxane=1/1) were added thereto at room temperature. The reaction solution was then transferred into an oil bath for reflux for 8 hours until the reaction was determined to be complete by TLC. The pH of the reaction solution was adjusted to neutral with dilute hydrochloric acid after cooling. After being extracted by EA for three times, EA phases were combined and washed with saturated salt water once, dried with anhydrous sodium sulfate, and then be evaporated followed by being recrystallized with PE/EA = 10/1 to obtain 6 g of yellow crystal, with a yield of 85%, and MS (ESI) (m/z): 233 $[M-H]^-$.

**3) Synthesis of 3-benzyloxy-N-(pyridin-3-yl)thiophene-2-carboxamide (compound 1-1), General Synthetic Method 1**

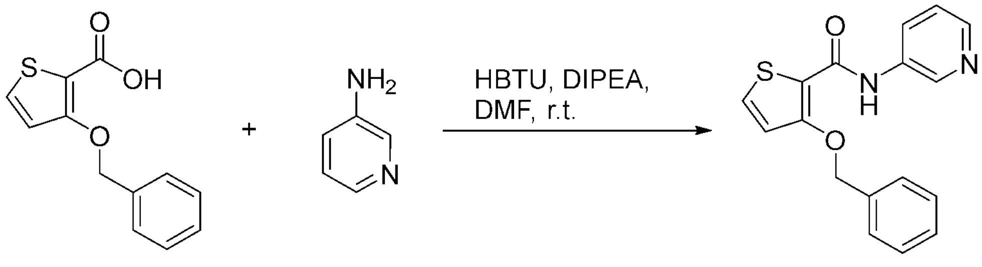


**5** **6** **1-1**

3 g (0.013mol, 1.0eq) of compound 5 and 7.3g (0.019mol, 1.5eq) of HBTU were weighed and placed into a 100 mL round bottom flask, and 50 mL of DMF were added thereto for dissolution. After 4.95 g (0.038 mol, 3.0 eq) of DIPEA were added while stirring, the reaction solution was stirred for 10 minutes at room temperature. After 3.6 g (0.038 mol, 3.0 eq) of compound 6 were added, the reaction solution was stirred overnight at room temperature until the reaction was determined to be complete by TLC. After being dissolved by EA, washed with saturated salt water for three times, dried with anhydrous sodium sulfate and purified by flash, 3.6 g of yellow solid was obtained, that is, 3-benzyloxy-N-(pyridin-3-yl) thiophene-2-carboxamide of formula 1-1, with a yield of 89%. MS(ESI) (m/z): 311[M+H]+. $^1$H NMR (400 MHz, DMSO-$d_6$) δ 9.47 (s, 1H), 8.58 (s, 1H), 8.28 (m, 1H), 8.04 (d, J = 8.2 Hz, 1H), 7.86 (d, J = 5.5 Hz, 1H), 7.59 (d, J = 7.1 Hz, 2H), 7.41 (m, 4H), 7.28 (d, J = 5.4 Hz, 1H), 5.44 (s, 2H).

**Example 2: Preparation of compounds 1-4, 1-5, 1-6, 1-7, 1-9, 1-14, 1-15, 1-20, 1-40, 1-61, 1-64, 1-65, 1-68, 1-69, and 1-83**

Referring to the conditions and steps of the General Synthetic Method 1 in Example 1, 3-benzyloxy-N-(pyridazin-3-yl)thiophene-2-carboxamide of Formula 1-4 was prepared by reacting pyridazin-3-amine with compound 5, with a yield of 50%. MS(ESI) (m/z): 312[M+H]+. $^1$H NMR (400 MHz, $CDCl_3$) δ 9.29 (d, 1H), 8.61 (d, 1H), 7.76 (d, 1H), 7.60 (t, 1H), 7.47 - 7.35 (m, 4H), 7.27 (s, 1H), 6.59 (m, 1H), 5.20 (s, 2H).

Referring to the conditions and steps of the General Synthetic Method 1 in Example 1, 3-benzyloxy-N-(4-(trifluoromethyl)pyridin-2-yl)thiophene-2-carboxamide of Formula 1-5 was prepared by reacting 2-amino-4-(trifluoromethyl)pyridine with compound 5, with a yield of 16%.MS(ESI) (m/z): 379[M+H]+. $^1$H NMR (400 MHz, $CDCl_3$) δ 9.97 (s, 1H), 8.64 (s, 1H), 8.43 (s, 1H), 7.53 - 7.33 (m, 5H), 7.27 (s, 1H), 7.23 (s, 1H), 6.91 (d, J = 4.5 Hz, 1H), 5.40 (s, 2H).

Referring to the conditions and steps of the General Synthetic Method 1 in Example 1, 3-benzyloxy-N-(4-chloropyridin-2-yl)thiophene-2-carboxamide of Formula 1-6 was prepared by reacting 2-amino-4-chloropyridine with compound 5, with a yield of 46%. MS(ESI) (m/z): 345, 347[M+H]+. $^1$H NMR (400 MHz, $CDCl_3$) δ 9.84 (s, 1H), 8.43 (s, 1H), 8.18 (s, 1H), 7.56 - 7.33 (m, 5H), 7.27 (s, 1H), 7.03 (s, 1H), 6.89 (s, 1H), 5.39 (s, 2H).

Referring to the conditions and steps of the General Synthetic Method 1 in Example 1, 3-benzyloxy-N-phenylthiophen-2-carboxamide of Formula 1-7 was prepared by reacting aniline with compound 5, with a yield of 68%. MS(ESI) (m/z): 310[M+H]+. $^1$H NMR (400 MHz, $CDCl_3$) δ 9.20 (s, 1H), 7.49 (m, 5H), 7.43 (m, 2H), 7.28 (s, 2H), 7.27 - 7.25 (m, 1H), 7.07 (s, 1H), 6.99 (s, 1H), 5.29 (s, 2H).

Referring to the conditions and steps of the General Synthetic Method 1 in Example 1, 3-benzyloxy-N-(6-hydroxypyridine-2-yl)thiophene-2-carboxamide of Formula 1-9 was prepared by reacting 6-amino-2-hydroxypyridine with compound 5, with a yield of 48%. MS(ESI) (m/z): 327[M+H]+. $^1$H NMR (400 MHz, $CDCl_3$) δ 9.53 (s, 1H), 7.76 (d, J = 7.5 Hz, 1H), 7.58 (t, J = 7.5 Hz, 1H), 7.43 (dd, J = 7.5, 1.5 Hz, 2H), 7.37 (m, 2H), 7.27 (m, 1H), 7.03 (dd, J = 7.5, 1.4 Hz, 1H), 6.68 - 6.51 (m, 2H), 6.51 - 6.45 (m, 1H), 5.20 (s, 2H).

Referring to the conditions and steps of the General Synthetic Method 1 in Example 1, 3-benzyloxy-5-nitro-N-(pyridin-3-yl)thiophene-2-carboxamide of Formula 1-14 was prepared by reacting 3-benzyloxy-5-nitrothiophene-2-carboxylic acid with compound 6, with a yield of 56%. MS(ESI) (m/z): 356[M+H]+. $^1$H NMR (400 MHz, $CDCl_3$) δ 9.16 (s, 1H), 8.34 (s, 1H), 8.20 (m, 2H), 7.78 (s, 1H), 7.50 (m, 2H), 7.25 (m, 4H), 5.32 (s, 2H).

Referring to the conditions and steps of the General Synthetic Method 1 in Example 1, 3-benzyloxy-4-bromo-N-(pyridin-3-yl)thiophene-2-carboxamide of Formula 1-15 was prepared by reacting 3-benzyloxy-4-bromothiophene-2-carboxylic acid with compound 6, with a yield of 32%. MS(ESI) (m/z): 388, 390[M+H]+. $^1$H NMR (400 MHz, $CDCl_3$) δ 9.03 (s, 1H), 8.29 (s, 1H), 7.99 (s, 2H), 7.55 - 7.39 (m, 5H), 7.24 (s, 1H), 7.19 (s, 1H), 5.35 (s, 2H).

Referring to the conditions and steps of the General Synthetic Method 1 in Example 1, 3-benzyloxy-N-(4,6-Dichloropyridin-2-yl)thiophene-2-carboxamide of Formula 1-20 was prepared by reacting 2-amino-4,6-dichloropyridine with compound 5, with a yield of 7%. MS(ESI) (m/z): 379, 381[M+H]+. $^1$H NMR (400 MHz, $CDCl_3$) δ 9.90 (s, 1H), 8.34 (s, 1H), 7.50 (d, J = 15.8 Hz, 5H), 7.41 (s, 1H), 7.28 (s, 1H), 7.06 (s, 1H), 6.94 (s, 1H), 5.38 (s, 2H).

Referring to the conditions and steps of the General Synthetic Method 1 in Example 1, 3-benzyloxy-5-methyl-N-(pyridin-3-yl)thiophene-2-carboxamide of Formula 1-40 was prepared by reacting 3-benzyloxy-5-methylthiophene-2-carboxylic acid with compound 6, with a yield of 55%. MS(ESI) (m/z): 325[M+H]+. $^1$H NMR (400 MHz, $CDCl_3$) δ 9.14 (s, 1H), 8.22 (d, J = 29.9 Hz, 3H), 7.48 (m, 5H), 7.20 (m, 1H), 6.71 (s, 1H), 5.23 (s, 2H), 2.51 (s, 3H).

Referring to the conditions and steps of the General Synthetic Method 1 in Example 1, 3-benzyloxy-N-(pyridin-2-yl)thiophene-2-carboxamide of Formula 1-61 was prepared by reacting 2-aminopyridine with compound 5, with a yield of 68%. MS(ESI) (m/z): 311[M+H]+. $^1$H NMR (400 MHz, $CDCl_3$) δ 9.78 (s, 1H), 8.31 (d, J = 7.8 Hz, 2H), 7.70 (s, 1H), 7.43 (m, 5H), 7.28 (s, 1H), 7.02 (s, 1H), 6.88 (s, 1H), 5.40 (s, 2H)

Referring to the conditions and steps of the General Synthetic Method 1 in Example 1, 3-benzyloxy-N-(pyrimidin-5-yl)thiophene-2-carboxamide of Formula 1-64 was prepared by reacting 5-aminopyrimidine with compound 5, with a yield of 43%. MS(ESI) (m/z): 312[M+H]+. $^1$H NMR (400 MHz, DMSO-$d_6$) δ 9.58 (s, 1H), 8.98 (m, 2H), 8.91 (s, 1H), 7.89 (s, 1H), 7.59 (m, 2H), 7.42 (m, 3H), 7.26 (s, 1H), 5.45 (s, 2H).

Referring to the conditions and steps of the General Synthetic Method 1 in Example 1, 3-benzyloxy-N-(thiazol-5-yl)thiophene-2-carboxamide of Formula 1-65 was prepared by reacting 5-thiazolamine with compound 5, with a yield of 48%. MS(ESI) (m/z): 317[M+H]+. $^1$H NMR (400 MHz, DMSO-$d_6$) δ 10.42 (s, 1H), 8.62 (s, 1H), 7.86 (s, 1H), 7.71 (s,1H), 7.55 (m, 2H), 7.40 (d, J = 24.8 Hz, 3H), 7.24 (s, 1H), 5.45 (s, 2H).

Referring to the conditions and steps of the General Synthetic Method 1 in Example 1, 3-benzyloxy-N-(2-methylpyridin-3-yl)thiophene-2-carboxamide of Formula 1-68 was prepared by reacting 3-amino-2-picoline with compound **5**, with a yield of 53%. MS(ESI) (m/z): 325[M+H]+. $^1$H NMR (400 MHz, DMSO-$d_6$) δ 9.05 (s, 1H), 8.37 (s, 1H), 8.15 (s, 1H), 7.89 (s, 1H), 7.59 (m, 2H), 7.43 (m, 3H), 7.34 (s, 1H), 7.21 (s, 1H), 5.42 (s, 2H), 1.96 (s, 3H).

Referring to the conditions and steps of the General Synthetic Method 1 in Example 1, 3-benzyloxy-N-(4-methylpyridin-3-yl)thiophene-2-carboxamide of Formula 1-69 was prepared by reacting 3-amino-4-methylpyridine with compound **5**, with a yield of 50%. MS(ESI) (m/z): 325[M+H]+. $^1$H NMR (400 MHz, $CDCl_3$) δ 9.38 (s, 1H), 8.84 (s, 1H), 8.21 (s, 1H), 7.55 - 7.42 (m, 6H), 7.00 (d, J = 11.6 Hz, 2H), 5.26 (s, 2H), 1.73 (s, 3H).

Referring to the conditions and steps of the General Synthetic Method 1 in Example 1, 3-benzyloxy-N-(5-methylpyridin-3-yl)thiophene-2-carboxamide of Formula 1-83 was prepared by reacting 5-methylpyridin-3-amine with compound **5**, with a yield of 72%. MS(ESI) (m/z): 325[M+H]+. $^1$H NMR (400 MHz, DMSO-$d_6$) δ 9.41 (s, 1H), 8.42 (s, 1H), 8.13 (s, 1H), 7.86 (s, 1H), 7.78 (s, 1H), 7.60 (m, 2H), 7.44 (s, 3H), 7.28 (s, 1H), 5.43 (s, 2H), 2.28 (s, 3H).

**Example 3: Preparation of 3-(2-chloro-5-fluorobenzyloxy)-N-(pyridin-3-yl)thiophene-2-carboxamide of formula 1-2**

**1) Synthesis of 3-hydroxy-N-(pyridin-3-yl)thiophene-2-carboxamide**

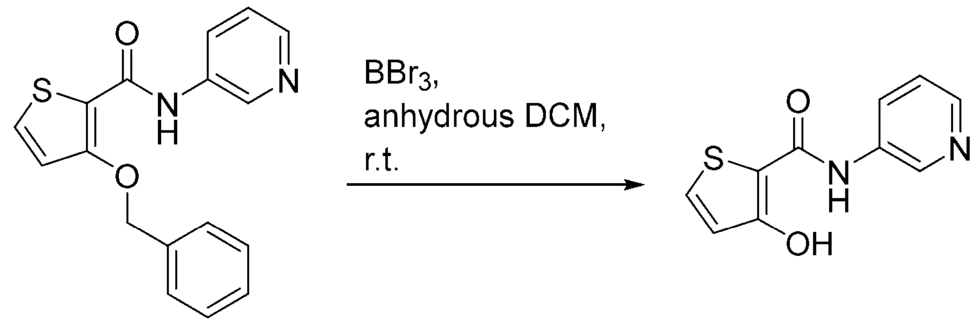


**1-1** **7**

2.3 g of compound 1-1 were weighed and placed into a 100 mL round bottom flask, and 30 mL of anhydrous dichloromethane were added thereto for dissolution. After 1 mL of boron tribromide were added while stirring, the reaction solution was stirred overnight at room temperature, meanwhile, a large amount of yellow solid was precipitated. Until the reaction was determined to be complete by TLC, an appropriate amount of methanol was added to quench and dissolve. After evaporating, an appropriate amount of water was added, the pH of the reaction solution was adjusted to alkaline with saturated sodium carbonate solution. After acidification with dilute hydrochloric acid, EA was added for extraction, the reaction solution was dried with anhydrous sodium sulfate, and then be evaporated to obtain 1 g of yellow solid, with a yield of 65%, and MS(ESI) (m/z): 221[M+H]+. The product can be used for the next reaction without further purification.

**2) Synthesis of 3-(2-chloro-5-fluorobenzyloxy)-N-(pyridin-3-yl)thiophene-2-carboxamide of formula 1-2**, **General Synthetic Method 2**

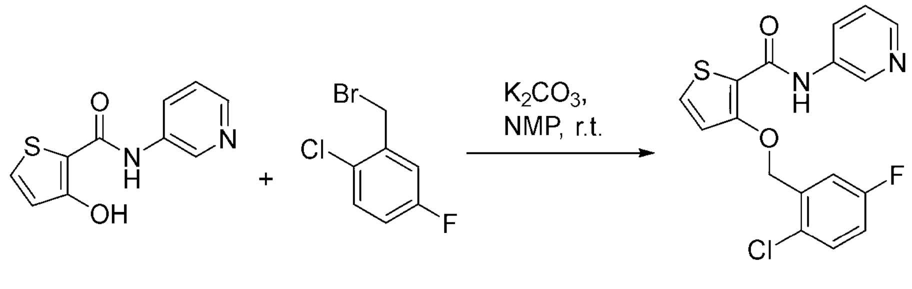


**7** **8** **1-2**

70mg (0.32mmol, 1.0eq) of compound 7 and 88mg (0.64mmol, 2.0eq) of potassium carbonate were weighed and placed into a round bottom flask, and 3 mL of NMP were added thereto for dissolution. After 92mg (0.413mmol, 1.3eq) of compound 8 were added while stirring, the reaction solution was stirred overnight at room temperature until the reaction was determined to be complete by TLC. After being dissolved by EA, washed with saturated salt water for three times, dried with anhydrous sodium sulfate and purified by flash, 75mg of white solid was obtained, that is, 3-(2-chloro-5-fluorobenzyloxy)-N-(pyridin-3-yl)thiophene-2-carboxamide of formula 1-2, with a yield of 65%. MS(ESI)(m/z): 363, 365[M+H]+. $^1H$ NMR (400 MHz, DMSO-$d_6$) $\delta$ 9.44 (s, 1H), 8.62 (s, 1H), 8.29 (m, 1H), 8.05 (s, 1H), 7.89 (s, 1H), 7.65 (m, 2H), 7.34 (m, 2H), 5.48 (s, 2H).

**Example 4: Preparation of compounds 1-3, 1-8, 1-10, 1-11, 1-12, 1-13, 1-16, 1-18, 1-19, 1-21, 1-22, 1-23, 1-25, 1-27, 1-28, 1-29, 1-30, 1-31, 1-32, 1-33, 1-34, 1-35, 1-36, 1-37, 1-38, 1-39, 1-41, 1-42, 1-43, 1-44, 1-45, 1-46, 1-47, 1-48, 1-49, 1-50, 1-51, 1-52, 1-53, 1-54, 1-55, 1-56, 1-57, 1-58, 1-59, 1-60, 1-63, 1-66, 1-67, 1-70, 1-71, 1-72, 1-73, 1-74, 1-75, 1-76, 1-77, 1-78, 1-79, 1-80, 1-81, 1-82, 1-84, 1-85, 1-86, 1-87, 1-88, 1-89, 1-93, 1-94, 1-97, 1-98, and 1-99**

Referring to the conditions and steps of the General Synthetic Method 2 in Example 3, 3-(2,6-dichlorobenzyloxy)-N-(pyridin-3-yl)thiophene-2-carboxamide of Formula 1-3 was prepared by reacting 2,6-dichlorobenzyl bromide with compound 7, with a yield of 75%. MS(ESI) (m/z):379, 381[M+H]+. $^1$H NMR (400 MHz, DMSO-$d_6$) δ 9.22 (s, 1H), 8.36 (d, J = 2.3 Hz, 1H), 8.27 (d, J = 4.6 Hz, 1H), 7.95 (d, J = 5.6 Hz, 2H), 7.64 (s, 1H), 7.62 (s, 1H), 7.53 (m, 1H), 7.45 (d, J = 5.5 Hz, 1H), 7.36 (dd, J = 8.2, 4.7 Hz, 1H), 5.60 (s, 2H).

Referring to the conditions and steps of the General Synthetic Method 2 in Example 3, 3-(5-fluoro-2-(2-hydroxy-2-methylpropoxy)benzyloxy)-N-(pyridin-3-yl)thiophene-2-carboxamide of Formula 1-8 was prepared by reacting 5-fluoro-2-(2-hydroxy-2-methylpropoxy)benzyl bromide with compound 7, with a yield of 44%. MS(ESI) (m/z): 416[M+H]+. $^1$H NMR (400 MHz, $CDCl_3$) δ 9.98 (s, 1H), 8.51 (d, J = 1.3 Hz, 1H), 8.24 (dd, J = 7.5, 1.3 Hz, 1H), 8.06 (d, J = 7.5 Hz, 1H), 7.77 (d, J = 7.3 Hz, 1H), 7.30 - 7.22 (m, 2H), 7.10 - 7.01 (m, 2H), 6.97 (m, 1H), 6.59 (d, J = 7.5 Hz, 1H), 5.21 (s, 2H), 4.25 (s, 2H), 2.19 (s, 1H), 1.35 (s, 6H).

Referring to the conditions and steps of the General Synthetic Method 2 in Example 3, 3-(naphthalen-1-ylmethoxy)-N-(pyridin-3-yl)thiophene-2-carboxamide of Formula 1-10 was prepared by reacting 1-(bromomethyl) naphthalene with compound 7, with a yield of 68%. MS(ESI) (m/z): 361[M+H]+. $^1$H NMR (400 MHz, DMSO-$d_6$) δ 9.28 (s, 1H), 8.35 (d, J = 7.5 Hz, 1H), 8.21 (s, 1H), 8.15 (s, 1H), 8.01 (s, 2H), 7.91 (s, 1H), 7.80 (d, J = 6.1 Hz, 1H), 7.73 (s, 1H), 7.61 - 7.44 (m, 4H), 7.27 (s, 1H), 5.93 (s, 2H).

Referring to the conditions and steps of the General Synthetic Method 2 in Example 3, 3-(naphthalen-2-ylmethoxy)-N-(pyridin-3-yl)thiophene-2-carboxamide of Formula 1-11 was prepared by reacting 2-(bromomethyl) naphthalene with compound 7, with a yield of 69%. MS(ESI) (m/z): 361[M+H]+. $^1$H NMR (400 MHz, DMSO-$d_6$) δ 9.53 (s, 1H), 8.61 (s, 1H), 8.25 (s, 1H), 8.14 - 7.79 (m, 6H), 7.71 (s, 1H), 7.52 (s, 2H), 7.30 (d, J = 11.6 Hz, 2H), 5.59 (s, 2H).

Referring to the conditions and steps of the General Synthetic Method 2 in Example 3, N-(pyridin-3-yl)-3-(quinolin-8-ylmethoxy)thiophene-2-carboxamide of Formula 1-12 was prepared by reacting 8-bromomethylquinoline with compound 7, with a yield of 78%. MS(ESI) (m/z): 362[M+H]+. $^1$H NMR (400 MHz, DMSO-$d_6$) δ 9.57 (s, 1H), 8.88 (s, 1H), 8.46 (d, J = 8.1 Hz, 1H), 8.27 (d, J = 14.2 Hz, 2H), 8.05 (d, J = 7.6 Hz, 2H), 7.86 (s, 2H), 7.68 (d, J = 7.1 Hz, 1H), 7.57 (d, J = 3.7 Hz, 1H), 7.43 (s, 1H), 7.29 (d, J = 24.7 Hz, 1H), 6.03 (s, 2H).

Referring to the conditions and steps of the General Synthetic Method 2 in Example 3, 3-(anthracen-9-ylmethoxy)-N-(pyridin-3-yl)thiophene-2-carboxamide of Formula 1-13 was prepared by reacting 9-(bromomethyl)anthracene with compound 7, with a yield of 55%. MS(ESI) (m/z): 411[M+H]+. $^1$H NMR (400 MHz, DMSO-$d_6$) δ 9.03 (s, 1H), 8.82 (s, 1H), 8.59 (d, J = 8.0 Hz, 2H), 8.21 (d, J = 8.0 Hz, 2H), 8.10 (s, 1H), 8.00 (s, 1H), 7.74 (s, 1H), 7.59 (s, 5H), 7.36 (d, J = 7.2 Hz, 1H), 7.13 (s, 1H), 6.50 (s, 2H).

Referring to the conditions and steps of the General Synthetic Method 2 in Example 3, 3-[(2-methoxynaphthalen-1-yl)methoxy]-N-(pyridin-3-yl)thiophene-2-carboxamide of Formula 1-16 was prepared by reacting 1-bromo-2-methoxynaphthalene with compound 7, with a yield of 88%. MS(ESI) (m/z): 391[M+H]+. $^1$H NMR (400 MHz, DMSO-$d_6$) δ 9.23 (s, 1H), 8.27 (d, J = 8.2 Hz, 1H), 8.19 (d, J = 3.9 Hz, 1H), 8.09 (d, J = 9.0 Hz, 1H), 8.02 (s, 1H), 7.93 (dd, J = 16.0, 6.8 Hz, 2H), 7.66 (d, J = 7.1 Hz, 1H), 7.56 (d, J = 9.0 Hz, 1H), 7.54 - 7.46 (m, 2H), 7.39 (d, J = 7.4 Hz, 1H), 7.26 (d, J = 4.0 Hz, 1H), 5.91 (s, 2H), 3.97 (s, 3H).

Referring to the conditions and steps of the General Synthetic Method 2 in Example 3, 6-(2-[(2-(pyridin-3-ylcarbamoyl)thiophene-3-yl)oxymethyl]phenoxy)ethyl hexanoate of Formula 1-18 was prepared by reacting 6-(2-(bromomethyl)phenoxy)ethyl hexanoate with compound 7, with a yield of 32%. MS(ESI) (m/z): 469[M+H]+. $^1$H NMR (400 MHz, DMSO-$d_6$) δ 9.43 (d, J = 14.0 Hz, 1H), 8.34 (d, J = 8.2 Hz, 1H), 8.25 (d, J = 11.2 Hz, 1H), 7.95 (d, J = 7.5 Hz, 1H), 7.88 (d, J = 10.8 Hz, 1H), 7.55 (s, 1H), 7.46 - 7.30 (m, 3H), 7.11 (d, J = 7.5 Hz, 1H), 7.01 (s, 1H), 5.37 (s, 2H), 3.95 (dd, J = 32.5, 10.5 Hz, 4H), 2.14 (s, 2H), 1.57 (s, 2H), 1.40 (s, 2H), 1.25 (s, 2H), 1.14 (t, J = 2.7 Hz, 3H).

Referring to the conditions and steps of the General Synthetic Method 2 in Example 3, N-(pyridin-3-yl)-3-[(6,7,8,9-tetrafluorodibenzo[b,e][1,4]dioxin-1-yl)methoxy]thiophene-2-carboxamide of Formula 1-19 was prepared by reacting (6,7,8,9-tetrafluorodibenzo[b,e][1,4]dioxin-1-yl)methyl bromide with compound 7, with a yield of 45%. MS(ESI) (m/z): 489[M+H]+. $^1$H NMR (400 MHz, DMSO-$d_6$) δ 9.41 (s, 1H), 8.52 (s, 1H), 8.21 (s, 1H), 7.99 (s, 1H), 7.90 (s, 1H), 7.39 - 7.26 (m, 3H), 7.22 (s, 1H), 7.17 (s, 1H), 5.43 (s, 2H).

Referring to the conditions and steps of the General Synthetic Method 2 in Example 3, 3-(2-nitrobenzyloxy)-N-(pyridin-3-yl)thiophene-2-carboxamide of Formula 1-21 was prepared by reacting 2-nitrobenzyl bromide with compound 7, with a yield of 16%. MS(ESI) (m/z): 356[M+H]+. $^1$H NMR (400 MHz, DMSO-$d_6$) δ 9.43 (s, 1H), 8.69 (s, 1H), 8.44 - 8.02 (m, 3H), 7.84 (m, 3H), 7.66 (s, 1H), 7.38 (s, 1H), 7.24 (s, 1H), 5.80 (s, 2H).

Referring to the conditions and steps of the General Synthetic Method 2 in Example 3, 2-[(2-(pyridin-3-ylcarbamoyl)thiophene-3-yl)oxymethyl]methyl benzoate of Formula 1-22 was prepared by reacting methyl 2-bromomethylbenzoate with compound **7**, with a yield of 16%. MS(ESI) (m/z): 369[M+H]+. $^1$H NMR (400 MHz, DMSO-$d_6$) δ 9.44 (s, 1H), 8.61 (s, 1H), 8.27 (s, 1H), 8.05 (d, J = 7.7 Hz, 1H), 7.97 (d, J = 7.5 Hz, 1H), 7.85 (d, J = 5.0 Hz, 1H), 7.75 (d, J = 7.4 Hz, 1H), 7.67 (t, J = 7.3 Hz, 1H), 7.54 (t, J = 7.2 Hz, 1H), 7.37 (s, 1H), 7.25 (d, J = 5.3 Hz, 1H), 5.72 (s, 2H), 3.74 (s, 3H).

Referring to the conditions and steps of the General Synthetic Method 2 in Example 3, trimethylacetate (4-chloro-3-((2-(pyridin-3-ylcarbamoyl)thiophene-3-yl)oxymethyl))benzoate of Formula **1-23** was prepared by reacting 2-chloro-5-(trimethylacetoxy)benzyl bromide with compound **7**, with a yield of 36%. MS(ESI) (m/z): 445, 447[M+H]+. $^1$H NMR (400 MHz, DMSO-$d_6$) δ 9.43 (s, 1H), 8.61 (d, J = 3.1 Hz, 1H), 8.26 (s, 1H), 8.00 (s, 1H), 7.88 (t, J = 5.6 Hz, 1H), 7.58 (dd, J = 12.6, 3.9 Hz, 2H), 7.33 (d, J = 5.5 Hz, 2H), 7.24 - 7.15 (m, 1H), 5.45 (s, 2H), 1.26 - 1.20 (s, 9H).

Referring to the conditions and steps of the General Synthetic Method 2 in Example 3, 4-chloro-3-[(2-(pyridin-3-ylcarbamoyl)thiophene-3-yl)oxymethyl]benzylphosphonate of Formula **1-25** was prepared by reacting 2-chloro-5-(diethoxyphosphoryloxy)benzyl bromide with compound **7**, with a yield of 85%. MS(ESI) (m/z): 497, 499[M+H]+. $^1$H NMR (400 MHz, DMSO-$d_6$) δ 9.45 (s, 1H), 8.64 (s, 1H), 8.29 (s, 1H), 8.04 (s, 1H), 7.89 (s, 1H), 7.59 (d, J = 10.6 Hz, 2H), 7.37 (s, 1H), 7.30 (t, J = 7.2 Hz, 2H), 5.50 (d, J = 8.7 Hz, 2H), 4.08 (dd, J = 15.2, 7.5 Hz, 4H), 1.22 - 1.14 (m, 6H).

Referring to the conditions and steps of the General Synthetic Method 2 in Example 3, 3-(2-(dodecyloxy)benzyloxy)-N-(pyridin-3-yl)thiophene-2-carboxamide of Formula 1-27 was prepared by reacting 2-(dodecyloxy)benzyl bromide with compound **7**, with a yield of 68%. MS(ESI)(m/z): 495[M+H]+. $^1$H NMR (400 MHz, $CDCl_3$) δ 9.39 (s, 1H), 8.26 (m, 2H), 8.15 (s, 1H), 7.46 (d, J = 34.6 Hz, 3H), 7.24 - 7.18 (m, 1H), 7.03 (s, 3H), 5.31 (s, 2H), 3.95 (m, 2H), 1.65 (m, 2H), 1.23 (m, 18H), 0.88 (t, 3H).

Referring to the conditions and steps of the General Synthetic Method 2 in Example 3, 3-(2-(undecyloxy)benzyloxy)-N-(pyridin-3-yl)thiophene-2-carboxamide of Formula 1-28 was prepared by reacting 2-(undecyloxy)benzyl bromide with compound 7, with a yield of 52%. MS(ESI) (m/z): 481[M+H]+. $^1$H NMR (400 MHz, $CDCl_3$) δ 9.39 (s, 1H), 8.28 (m, 2H), 8.15 (s, 1H), 7.47 (d, J = 33.6 Hz, 3H), 7.26 - 7.17 (m, 1H), 7.04 (m, 3H), 5.42 (s, 2H), 3.96 (m, 2H), 1.66 (m, 2H), 1.22 (m, 16H), 0.88 (t, 3H).

Referring to the conditions and steps of the General Synthetic Method 2 in Example 3, 3-(2-(decyloxy)benzyloxy)-N-(pyridin-3-yl)thiophene-2-carboxamide of Formula 1-29 was prepared by reacting 2-(decyloxy)benzyl bromide with compound **7**, with a yield of 48%. MS(ESI) (m/z): 467[M+H]+. $^1$H NMR (400 MHz, $CDCl_3$) δ 9.43 (d, J = 18.0 Hz, 1H), 8.31 (d, J = 18.7 Hz, 2H), 8.19 (d, J = 18.3 Hz, 1H), 7.60 - 7.40 (m, 3H), 7.28 (d, J = 5.6 Hz, 2H), 7.08 (d, J = 18.7 Hz, 3H), 5.32 (s, 2H), 3.98 (m, 2H), 1.68 (m, 2H), 1.24 (m, 14H), 0.92 (t, 3H).

Referring to the conditions and steps of the General Synthetic Method 2 in Example 3, 3-(2-(nonyloxy)benzyloxy)-N-(pyridin-3-yl)thiophene-2-carboxamide of Formula 1-30 was prepared by reacting 2-(nonyloxy)benzyl bromide with compound **7**, with a yield of 58%. MS(ESI) (m/z): 453[M+H]+. $^1$H NMR (400 MHz, $CDCl_3$) δ 9.38 (s, 1H), 8.27 (m, 2H), 8.15 (s, 1H), 7.50 (s, 1H), 7.42 (m, 2H), 7.25 (s, 1H), 7.23 (s, 1H), 7.08 - 6.96 (m, 3H), 5.31 (s, 2H), 3.95 (d, J = 4.9 Hz, 2H), 1.65 (m, 2H), 1.22 (m, 12H), 0.86 (t, 3H).

Referring to the conditions and steps of the General Synthetic Method 2 in Example 3, 3-(2-(octyloxy)benzyloxy)-N-(pyridin-3-yl)thiophene-2-carboxamide of Formula 1-31 was prepared by reacting 2-(octyloxy)benzyl bromide with compound 7, with a yield of 59%. MS(ESI) (m/z): 439[M+H]+. $^1$H NMR (400 MHz, $CDCl_3$) δ 9.38 (s, 1H), 8.27 (s, 2H), 8.15 (s, 1H), 7.50 (s, 1H), 7.42 (s, 2H), 7.25 - 7.16 (m, 1H), 7.08 - 6.95 (m, 3H), 5.31 (s, 2H), 3.96 (s, 2H), 1.65 (d, J = 5.9 Hz, 2H), 1.35 - 1.13 (m, 10H), 0.86 (t, J = 6.7 Hz, 3H).

Referring to the conditions and steps of the General Synthetic Method 2 in Example 3, 3-(2-(heptyloxy)benzyloxy)-N-(pyridin-3-yl)thiophene-2-carboxamide of Formula 1-32 was prepared by reacting 2-(heptyloxy)benzyl bromide with compound **7**, with a yield of 52%. MS(ESI) (m/z): 425[M+H]+. $^1$H NMR (400 MHz, $CDCl_3$) δ 9.38 (s, 1H), 8.26 (d, J = 8.2 Hz, 2H), 8.15 (s, 1H), 7.51 (d, J = 5.0 Hz, 1H), 7.42 (m, 2H), 7.23 (s, 1H), 7.02 (dd, J = 22.1, 7.0 Hz, 3H), 5.29 (s, 2H), 3.96 (t, J = 6.1 Hz, 2H), 1.70 - 1.61 (m, 2H), 1.38 - 1.11 (m, 8H), 0.83 (t, 3H).

Referring to the conditions and steps of the General Synthetic Method 2 in Example 3, 3-(2-(hexyloxy)benzyloxy)-N-(pyridin-3-yl)thiophene-2-carboxamide of Formula 1-33 was prepared by reacting 2-(hexyloxy)benzyl bromide with compound **7**, with a yield of 56%. MS(ESI) (m/z): 411[M+H]+. $^1$H NMR (400 MHz, $CDCl_3$) δ 9.39 (s, 1H), 8.27 (m, 2H), 8.15 (s, 1H), 7.51 (s, 1H), 7.42 (m, 2H), 7.24 (m, 2H), 7.05 (m, 3H), 5.32 (s, 2H), 3.96 (m, 2H), 1.65 (m, 2H), 1.25 (m, 6H), 0.81 (t, 3H).

Referring to the conditions and steps of the General Synthetic Method 2 in Example 3, 3-(2-(pentyloxy)benzyloxy)-N-(pyridin-3-yl)thiophene-2-carboxamide of Formula 1-34 was prepared by reacting 2-(pentyloxy)benzyl bromide with compound **7**, with a yield of 21%. MS(ESI) (m/z): 397[M+H]+. $^1$H NMR (400 MHz, $CDCl_3$) δ 9.38 (s, 1H), 8.27 (m, 2H), 8.15 (s,1H), 7.51 (s, 1H), 7.42 (m, 2H), 7.24 - 7.19 (m, 1H), 7.02 (d, J = 18.8 Hz, 3H), 5.32 (s, 2H), 3.96 (m, 2H), 1.65 (m, 2H), 1.26 (m, 4H), 0.81 (t, 3H).

Referring to the conditions and steps of the General Synthetic Method 2 in Example 3, 3-(2-(butoxy)benzyloxy)-N-(pyridin-3-yl)thiophene-2-carboxamide of Formula 1-35 was prepared by reacting 2-(butoxy)benzyl bromide with compound **7**, with a yield of 30%. MS(ESI) (m/z): 383[M+H]+. $^1$H NMR (400 MHz, $CDCl_3$) δ 9.38 (s, 1H), 8.27 (s, 2H), 8.16 (s, 1H), 7.52 (s, 1H), 7.43 (2, 2H), 7.25 - 7.18 (m, 1H), 7.02 (d, J = 20.3 Hz, 3H), 5.37 (s, 2H), 3.97 (m, 2H), 1.65 (m, 2H), 1.35

(m, 2H), 0.84 (t, 3H).

Referring to the conditions and steps of the General Synthetic Method 2 in Example 3, 3-(2-(propoxy)benzyloxy)-N-(pyridin-3-yl)thiophene-2-carboxamide of Formula 1-36 was prepared by reacting 2-(propoxy)benzyl bromide with compound **7**, with a yield of 91%. MS(ESI) (m/z): 369[M+H]+. $^1$H NMR (400 MHz, $CDCl_3$) δ 9.39 (s, 1H), 8.27 (s, 2H), 8.17 (s, 1H), 7.52 (s,1H), 7.42 (m, 2H), 7.25 - 7.16 (m, 1H), 7.03 (d, J = 22.0 Hz, 3H), 5.33 (s, 2H), 3.93 (m, 2H), 1.69 (m, 2H), 0.91 (t, 3H).

Referring to the conditions and steps of the General Synthetic Method 2 in Example 3, 3-(2-(ethoxy)benzyloxy)-N-(pyridin-3-yl)thiophene-2-carboxamide of Formula **1-37** was prepared by reacting 2-(ethoxy)benzyl bromide with compound **7**, with a yield of 57%. MS(ESI) (m/z): 355[M+H]+. $^1$H NMR (400 MHz, $CDCl_3$) δ 9.40 (s, 1H), 8.27 (s, 2H), 8.19 (s, 1H), 7.52 (s, 1H), 7.42 (d, J = 6.7 Hz, 2H), 7.25 - 7.18 (m, 1H), 7.11 - 6.90 (m, 3H), 5.38 (s, 2H), 4.04 (m, 2H), 1.31 (t, 3H).

Referring to the conditions and steps of the General Synthetic Method 2 in Example 3, 3-(2-(n-pentyloxy)-6-chlorobenzyloxy)-N-(pyridin-3-yl)thiophene-2-carboxamide of Formula **1-38** was prepared by reacting 2-(n-pentyloxy)-6-chlorobenzyl bromide with compound **7**, with a yield of 57%. MS(ESI) (m/z): 430, 432[M+H]+. $^1$H NMR (400 MHz, $CDCl_3$) δ 9.32 (s, 1H), 8.27 (d, J = 2.8 Hz, 2H), 8.17 (d, J = 1.8 Hz, 1H), 7.51 (d, J = 5.4 Hz, 1H), 7.34 (d, J = 8.2 Hz, 1H), 7.24 (d, J = 7.8 Hz, 1H), 7.13 (dd, J = 16.8, 6.7 Hz, 2H), 6.90 (d, J = 8.1 Hz, 1H), 5.50 (s, 2H), 3.97 (m, 2H), 1.71 (m, 2H), 1.30 (m,4H), 0.84 (t, 3H).

Referring to the conditions and steps of the General Synthetic Method 2 in Example 3, 3-(2-chloro-6-propoxybenzyloxy)-N-(pyridin-3-yl)thiophene-2-carboxamide of Formula 1-39 was prepared by reacting 2-chloro-6-propoxybenzyl bromide with compound 7, with a yield of 52%. MS(ESI) (m/z): 403, 405[M+H]+. $^1$H NMR (400 MHz, $CDCl_3$) δ 9.32 (s, 1H), 8.28 (m, 2H), 8.18 (s, 1H), 7.51 (s, 1H), 7.34 (s, 1H), 7.25 - 7.20 (m, 1H), 7.14 (d, J = 15.8 Hz, 2H), 6.90 (s, 1H), 5.51 (s, 2H), 3.94 (m, 2H), 1.75 (m, 2H), 0.94 (t, 3H).

Referring to the conditions and steps of the General Synthetic Method 2 in Example 3, 3-(2-methylbenzyloxy)-N-(pyridin-3-yl)thiophene-2-carboxamide of Formula 1-41 was prepared by reacting 2-methylbenzyl bromide with compound 7, with a yield of 91%. MS(ESI) (m/z): 325[M+H]+. $^1$H NMR (400 MHz, DMSO-$d_6$) δ 9.44 (s, 1H), 8.48 (s, 1H), 8.28 (s, 1H), 8.00 (d, J = 7.0 Hz, 1H), 7.89 (s, 1H), 7.54 (d, J = 6.3 Hz, 1H), 7.30 (dd, J = 21.5, 7.0 Hz, 5H), 5.46 (s, 2H), 2.40 (s, 3H).

Referring to the conditions and steps of the General Synthetic Method 2 in Example 3, 3-(4-methylbenzyloxy)-N-(pyridin-3-yl)thiophene-2-carboxamide of Formula 1-42 was prepared by reacting 4-methylbenzyl bromide with compound 7, with a yield of 60%. MS(ESI) (m/z): 325[M+H]+. $^1$H NMR (400 MHz, DMSO-$d_6$) δ 9.46 (s, 1H), 8.58 (s, 1H), 8.29 (s, 1H), 8.04 (d, J = 7.1 Hz, 1H), 7.86 (d, J = 5.3 Hz, 1H), 7.48 (d, J = 7.4 Hz, 2H), 7.38 (d, J = 5.3 Hz, 1H), 7.26 (dd, J = 15.0, 6.2 Hz, 3H), 5.39 (s, 2H), 2.31 (s, 3H).

Referring to the conditions and steps of the General Synthetic Method 2 in Example 3, 3-(3-methylbenzyloxy)-N-(pyridin-3-yl)thiophene-2-carboxamide of Formula 1-43 was prepared by reacting 3-methylbenzyl bromide with compound 7, with a yield of 84%. MS(ESI) (m/z): 325[M+H]+. $^1$H NMR (400 MHz, DMSO-$d_6$) δ 9.49 (s, 1H), 8.58 (s, 1H), 8.28 (d, J = 4.7 Hz, 1H), 8.05 (d, J = 8.3 Hz, 1H), 7.87 (d, J = 5.5 Hz, 1H), 7.43 (s, 1H), 7.33 (ddd, J = 27.2, 12.9, 6.4 Hz, 4H), 7.20 (d, J = 7.0 Hz, 1H), 5.39 (s, 2H), 2.31 (s, 3H).

Referring to the conditions and steps of the General Synthetic Method 2 in Example 3, 3-(2-fluorobenzyloxy)-N-(pyridin-3-yl)thiophene-2-carboxamide of Formula 1-44 was prepared by reacting 2-fluorobenzyl bromide with compound 7, with a yield of 67%. MS(ESI) (m/z): 329[M+H]+. $^1$H NMR (400 MHz, DMSO-$d_6$) δ 9.42 (s, 1H), 8.57 (s, 1H), 8.29 (d, J = 4.4 Hz, 1H), 8.03 (d, J = 8.0 Hz, 1H), 7.89 (d, J = 5.5 Hz, 1H), 7.70 (t, J = 7.4 Hz, 1H), 7.58 - 7.43 (m, 1H), 7.44 - 7.23 (m, 4H), 5.52 (s, 2H).

Referring to the conditions and steps of the General Synthetic Method 2 in Example 3, 3-(3-fluorobenzyloxy)-N-(pyridin-3-yl)thiophene-2-carboxamide of Formula 1-45 was prepared by reacting 3-fluorobenzyl bromide with compound 7, with a yield of 54%. MS(ESI) (m/z): 329[M+H]+. $^1$H NMR (400 MHz, DMSO-$d_6$) δ 9.34 (s, 1H), 8.50 (s, 1H), 8.15 (s, 1H), 7.94 (s, 1H), 7.72 (s, 1H), 7.31 (d, J = 22.9 Hz, 4H), 7.10 (m, 2H), 5.31 (d, J = 7.5 Hz, 2H).

Referring to the conditions and steps of the General Synthetic Method 2 in Example 3, 3-(4-fluorobenzyloxy)-N-(pyridin-3-yl)thiophene-2-carboxamide of Formula 1-46 was prepared by reacting 4-fluorobenzyl bromide with compound 7, with a yield of 41%. MS(ESI) (m/z): 329[M+H]+. $^1$H NMR (400 MHz, DMSO-$d_6$) δ 9.45 (s, 1H), 8.60 (s, 1H), 8.29 (s, 1H), 8.04 (d, J = 7.4 Hz, 1H), 7.86 (d, J = 5.2 Hz, 1H), 7.67 (m, 2H), 7.38 (d, J = 4.7 Hz, 1H), 7.27 (t, J = 7.5 Hz, 3H), 5.42 (s, 2H).

Referring to the conditions and steps of the General Synthetic Method 2 in Example 3, 3-(2-chlorobenzyloxy)-N-(pyridin-3-yl)thiophene-2-carboxamide of Formula 1-47 was prepared by reacting 2-chlorobenzyl bromide with compound 7, with a yield of 48%. MS(ESI) (m/z): 345, 347[M+H]+. $^1$H NMR (400 MHz, DMSO-$d_6$) δ 9.42 (s, 1H), 8.55 (s, 1H), 8.29 (d, J = 4.1 Hz, 1H), 8.03 (d, J = 8.1 Hz, 1H), 7.90 (d, J = 5.5 Hz, 1H), 7.74 (d, J = 6.8 Hz, 1H), 7.58 (d, J = 7.3 Hz, 1H), 7.51 - 7.30 (m, 4H), 5.52 (s, 2H).

Referring to the conditions and steps of the General Synthetic Method 2 in Example 3, 3-(3-chlorobenzyloxy)-N-(pyridin-3-yl)thiophene-2-carboxamide of Formula 1-48 was prepared by reacting 3-chlorobenzyl bromide with compound 7, with a yield of 56%. MS(ESI) (m/z): 345, 347[M+H]+. $^1$H NMR (400 MHz, DMSO-$d_6$) δ 9.49 (s, 1H), 8.67 (s,

1H), 8.29 (s, 1H), 8.07 (d, J = 8.2 Hz, 1H), 7.87 (d, J = 5.5 Hz, 1H), 7.73 (s, 1H), 7.54 (s, 1H), 7.51 - 7.42 (m, 2H), 7.41 - 7.32 (m, 1H), 7.25 (d, J = 5.3 Hz, 1H), 5.44 (s, 2H).

Referring to the conditions and steps of the General Synthetic Method 2 in Example 3, 3-(4-chlorobenzyloxy)-N-(pyridin-3-yl)thiophene-2-carboxamide of Formula 1-49 was prepared by reacting 4-chlorobenzyl bromide with compound 7, with a yield of 63%. MS(ESI) (m/z): 345, 347[M+H]+. $^1$H NMR (400 MHz, DMSO-$d_6$) δ 9.46 (s, 1H), 8.63 (s, 1H), 8.29 (d, J = 4.2 Hz, 1H), 8.06 (d, J = 8.3 Hz, 1H), 7.86 (d, J = 5.5 Hz, 1H), 7.62 (d, J = 8.1 Hz, 2H), 7.50 (d, J = 8.1 Hz, 2H), 7.44 - 7.35 (m, 1H), 7.25 (d, J = 5.5 Hz, 1H), 5.43 (s, 2H).

Referring to the conditions and steps of the General Synthetic Method 2 in Example 3, 3-(2-cyanobenzyloxy)-N-(pyridin-3-yl)thiophene-2-carboxamide of Formula 1-50 was prepared by reacting 2-cyanobenzyl bromide with compound 7, with a yield of 51%. MS(ESI) (m/z): 336[M+H]+. $^1$H NMR (400 MHz, $CDCl_3$) δ 9.01 (s, 1H), 8.32 (d, J = 13.9 Hz, 3H), 7.65 (m, 5H), 7.25 (m, 1H), 7.00 (s, 1H), 5.50 (s, 2H).

Referring to the conditions and steps of the General Synthetic Method 2 in Example 3, 3-(3-cyanobenzyloxy)-N-(pyridin-3-yl)thiophene-2-carboxamide of Formula 1-51 was prepared by reacting 3-cyanobenzyl bromide with compound 7, with a yield of 54%. MS(ESI) (m/z): 336[M+H]+. $^1$H NMR (400 MHz, $CDCl_3$) δ 9.01 (s, 1H), 8.31 (m, 2H), 8.23 (s, 1H), 7.77 (m, 3H), 7.63 (s, 1H), 7.53 (s, 1H), 6.93 (s, 1H), 5.34 (s, 2H).

Referring to the conditions and steps of the General Synthetic Method 2 in Example 3, 3-(4-cyanobenzyloxy)-N-(pyridin-3-yl)thiophene-2-carboxamide of Formula 1-52 was prepared by reacting 4-cyanobenzyl bromide with compound 7, with a yield of 54%. MS(ESI) (m/z): 336[M+H]+. $^1$H NMR (400 MHz, $CDCl_3$) δ 9.02 (s,1H), 8.34 (d, J = 14.2 Hz, 2H), 8.23 (s, 1H), 7.78 (d, J = 8.0 Hz, 2H), 7.61 (d, J = 7.3 Hz, 2H), 7.52 (d, J = 5.4 Hz, 1H), 7.27 (s, 1H), 6.90 (d, J = 5.5 Hz, 1H), 5.38 (s, 2H).

Referring to the conditions and steps of the General Synthetic Method 2 in Example 3, 3-(2-(trifluoromethoxy) benzyloxy)-N-(pyridin-3-yl)thiophene-2-carboxamide of Formula 1-53 was prepared by reacting 2-(trifluoromethoxy) benzyl bromide with compound 7, with a yield of 51%. MS(ESI) (m/z): 395[M+H]+. $^1$H NMR (400 MHz, $CDCl_3$) δ 9.09 (s, 1H), 8.27 (m, 1H), 7.60 (d, J = 7.3 Hz, 3H), 7.52 (s, 1H), 7.41 (m, 2H), 7.29 (m, 2H), 7.00 (s, 1H), 5.39 (s, 2H).

Referring to the conditions and steps of the General Synthetic Method 2 in Example 3, 3-(4-(trifluoromethoxy) benzyloxy)-N-(pyridin-3-yl)thiophene-2-carboxamide of Formula 1-54 was prepared by reacting 4-(trifluoromethoxy) benzyl bromide with compound 7, with a yield of 70%. MS(ESI) (m/z): 395[M+H]+. $^1$H NMR (400 MHz, $CDCl_3$) δ 9.12 (s, 1H), 8.41 (s, 1H), 8.32 (s, 1H), 8.13 (s, 1H), 7.53 (m, 3H), 7.26 (m, 3H), 6.96 (s, 1H), 5.30 (s, 2H).

Referring to the conditions and steps of the General Synthetic Method 2 in Example 3, 3-(3-(trifluoromethoxy) benzyloxy)-N-(pyridin-3-yl)thiophene-2-carboxamide of Formula 1-55 was prepared by reacting 3-(trifluoromethoxy) benzyl bromide with compound 7, with a yield of 55%. MS(ESI) (m/z): 395[M+H]+. $^1$H NMR (400 MHz, $CDCl_3$) δ 9.10 (s, 1H), 8.32 (m, 2H), 8.18 (s, 1H), 7.49 (d, J = 29.7 Hz, 3H), 7.34 (m, 2H), 7.23 (s, 1H), 6.96 (s, 1H), 5.31 (s, 2H).

Referring to the conditions and steps of the General Synthetic Method 2 in Example 3, 3-(4-methoxybenzyloxy)-N-(pyridin-3-yl)thiophene-2-carboxamide of Formula 1-56 was prepared by reacting 4-methoxybenzyl bromide with compound 7, with a yield of 23%. MS(ESI) (m/z): 341[M+H]+. $^1$H NMR (400 MHz, $CDCl_3$) δ 9.24 (s, 1H), 8.26 (d, J = 17.2 Hz, 3H), 7.51 (s, 1H), 7.41 (s, 2H), 7.20 (m, 1H), 6.99 (s, 3H), 5.21 (s, 2H), 3.85 (s, 3H).

Referring to the conditions and steps of the General Synthetic Method 2 in Example 3, 3-(2-methoxybenzyloxy)-N-(pyridin-3-yl)thiophene-2-carboxamide of Formula 1-57 was prepared by reacting 2-methoxybenzyl bromide with compound 7, with a yield of 78%. MS(ESI) (m/z): 341[M+H]+. $^1$H NMR (400 MHz, $CDCl_3$) δ 9.39 (s, 1H), 8.28 (s, 1H), 8.22 (m, 2H), 7.51 (s, 1H), 7.42 (s, 1H), 7.23 (m, 2H), 7.02 (dd, J = 20.4, 6.7 Hz, 3H), 5.32 (s, 2H), 3.82 (s, 3H).

Referring to the conditions and steps of the General Synthetic Method 2 in Example 3, 3-(3-methoxybenzyloxy)-N-(pyridin-3-yl)thiophene-2-carboxamide of Formula 1-58 was prepared by reacting 3-methoxybenzyl bromide with compound 7, with a yield of 46%. MS(ESI) (m/z): 341[M+H]+. $^1$H NMR (400 MHz, $CDCl_3$) δ 9.23 (s, 1H), 8.29 (m, 3H), 7.45 (m, 2H), 7.29 (s, 1H), 7.00 (m, 4H), 5.26 (s, 2H), 3.83 (s, 3H).

Referring to the conditions and steps of the General Synthetic Method 2 in Example 3, 3-(2-(trifluoromethyl) benzyloxy)-N-(pyridin-3-yl)thiophene-2-carboxamide of Formula 1-59 was prepared by reacting 2-(trifluoromethyl)benzyl bromide with compound 7, with a yield of 48%. MS(ESI) (m/z): 379[M+H]+. $^1$H NMR (400 MHz, $CDCl_3$) δ 9.09 (s, 1H), 8.26 (m, 3H), 7.66 (dd, J = 81.3, 36.6 Hz, 5H), 7.18 (m, 1H), 6.97 (s, 1H), 5.51 (s, 2H).

Referring to the conditions and steps of the General Synthetic Method 2 in Example 3, 3-(2,6-dimethylbenzyloxy)-N-(pyridin-3-yl)thiophene-2-carboxamide of Formula 1-60 was prepared by reacting 2,6-dimethylbenzyl bromide with compound 7, with a yield of 55%. MS(ESI) (m/z): 339[M+H]+. $^1$H NMR (400 MHz, $CDCl_3$) δ 9.19 (s, 1H), 8.24 (s, 1H), 8.11 (s, 1H), 7.91 (s, 1H), 7.56 (s, 1H), 7.28 (s, 1H), 7.18 (m, 3H), 7.10 (s, 1H), 5.34 (s, 2H), 2.46 (s, 6H).

Referring to the conditions and steps of the General Synthetic Method 2 in Example 3, 3-(2-chloro-6-methoxybenzyloxy)-N-(pyridin-3-yl)thiophene-2-carboxamide of Formula 1-63 was prepared by reacting 2-chloro-6-methoxybenzyl bromide with compound 7, with a yield of 45%. MS(ESI) (m/z): 375, 377[M+H]+. $^1$H NMR (400 MHz, $CDCl_3$) δ 9.37 (s, 1H), 8.32 (m, 2H), 8.26 (s, 1H), 7.55 (s, 1H), 7.41 (s, 1H), 7.27 (m, 1H), 7.18 (d, J = 8.0 Hz, 2H), 6.96 (d, J = 7.9 Hz, 1H), 5.55 (s, 2H), 3.90 (s, 3H).

Referring to the conditions and steps of the General Synthetic Method 2 in Example 3, 3-(benzo[b]thiophen-3-ylmethoxy)-N-(pyridin-3-yl)thiophene-2-carboxamide of Formula 1-66 was prepared by reacting 3-(bromomethyl)benzo[b]thiophene with compound 7, with a yield of 34%. MS(ESI) (m/z): 367[M+H]+. $^1H$ NMR (400 MHz, $CDCl_3$) δ 8.99 (s, 1H), 8.20 (s, 1H), 7.98 (s, 1H), 7.88 (d, J = 8.8 Hz, 2H), 7.76 (s, 1H), 7.63 (s, 1H), 7.56 (s, 1H), 7.40 (m, 2H), 7.09 (m, 2H), 5.53 (s, 2H).

Referring to the conditions and steps of the General Synthetic Method 2 in Example 3, 3-(2,3,5,6-tetrafluorobenzyloxy)-N-(pyridin-3-yl)thiophene-2-carboxamide of Formula 1-67 was prepared by reacting 2,3,5,6-tetrafluorobenzyl bromide with compound 7, with a yield of 65%. MS(ESI) (m/z): 383[M+H]+. $^1H$ NMR (400 MHz, $CDCl_3$) δ 8.98 (s, 1H), 8.49 (s, 1H), 8.35 (d, J = 3.6 Hz, 1H), 8.26 (d, J = 8.2 Hz, 1H), 7.54 (d, J = 5.5 Hz, 1H), 7.29 (d, J = 8.0 Hz, 1H), 7.23 - 7.14 (m, 1H), 7.07 (d, J = 5.5 Hz, 1H), 5.49 (s, 2H).

Referring to the conditions and steps of the General Synthetic Method 2 in Example 3, 3-(2-(phenylethoxy)benzyloxy)-N-(pyridin-3-yl)thiophene-2-carboxamide of Formula 1-70 was prepared by reacting 2-(phenylethoxy)benzyl bromide with compound 7, with a yield of 80%. MS(ESI) (m/z): 431[M+H]+. $^1H$ NMR (400 MHz, $CDCl_3$) δ 9.30 (s, 1H), 8.27 (m, 2H), 8.11 (s, 1H), 7.52 (s, 1H), 7.40 (m, 2H), 7.25 - 7.19 (m, 1H), 7.05 (dd, J = 45.4, 17.6 Hz, 8H), 5.25 (s, 2H), 4.20 (t, 2H), 2.96 (t, 2H).

Referring to the conditions and steps of the General Synthetic Method 2 in Example 3, 3-(4-chloro-2-(hexyloxy)benzyloxy)-N-(pyridin-3-yl)thiophene-2-carboxamide of Formula 1-71 was prepared by reacting 4-chloro-2-(hexyloxy)benzyl bromide with compound 7, with a yield of 60%. MS(ESI) (m/z): 445, 447[M+H]+. $^1H$ NMR (400 MHz, $CDCl_3$) δ 9.27 (s, 1H), 8.28 (d, J = 12.0 Hz, 3H), 7.51 (s, 1H), 7.45 - 7.32 (m, 2H), 7.26 - 7.17 (m, 1H), 7.00 (s, 1H), 6.92 (d, J = 8.6 Hz, 1H), 5.26 (s, 2H), 3.94 (m, 2H), 1.66 (m, 2H), 1.25 (m, 6H), 0.82 (t, 3H).

Referring to the conditions and steps of the General Synthetic Method 2 in Example 3, 3-(4-chloro-2-(heptyloxy)benzyloxy)-N-(pyridin-3-yl)thiophene-2-carboxamide of Formula 1-72 was prepared by reacting 4-chloro-2-(heptyloxy)benzyl bromide with compound 7, with a yield of 70%. MS(ESI) (m/z): 459, 461[M+H]+. $^1H$ NMR (400 MHz, $CDCl_3$) δ 9.25 (s, 1H), 8.26 (d, J = 7.6 Hz, 3H), 7.51 (s, 1H), 7.38 (d, J = 15.2 Hz, 2H), 7.25 (s, 2H), 7.00 (s, 1H), 6.90 (d, J = 7.7 Hz, 1H), 5.26 (s, 2H), 3.93 (m, 2H), 1.65 (m, 2H), 1.23 (m, 8H), 0.83 (t, 3H).

Referring to the conditions and steps of the General Synthetic Method 2 in Example 3, 3-(5-chloro-2-(hexyloxy)benzyloxy)-N-(pyridin-3-yl)thiophene-2-carboxamide of Formula 1-73 was prepared by reacting 5-chloro-2-(hexyloxy)benzyl bromide with compound 7, with a yield of 15%. MS(ESI) (m/z): 445, 447[M+H]+. $^1H$ NMR (400 MHz, $CDCl_3$) δ 9.27 (s, 1H), 8.28 (d, J = 10.5 Hz 3H), 7.52 (s, 1H), 7.40 (d, J = 14.8 Hz, 3H), 7.01 (s, 1H), 6.93 (s, 1H), 5.26 (s, 2H), 3.95 (m, 2H), 1.66 (m, 2H), 1.26 (m, 6H), 0.82 (t, 3H).

Referring to the conditions and steps of the General Synthetic Method 2 in Example 3, 3-(5-chloro-2-(heptyloxy)benzyloxy)-N-(pyridin-3-yl)thiophene-2-carboxamide of Formula 1-74 was prepared by reacting 5-chloro-2-(heptyloxy)benzyl bromide with compound 7, with a yield of 56%. MS(ESI) (m/z): 459, 461[M+H]+. $^1H$ NMR (400 MHz, $CDCl_3$) δ 9.07 (s, 1H), 8.07 (m, 3H), 7.32 (s, 1H), 7.22 (m, 2H), 7.08 (s, 1H), 6.81 (s, 1H), 6.73 (s, 1H), 5.07 (s, 2H), 3.74 (m, 2H), 1.47 (m, 2H), 1.05 (m, 8H), 0.65 (t, 3H).

Referring to the conditions and steps of the General Synthetic Method 2 in Example 3, 3-(2-(4-chlorobutoxy)benzyloxy)-N-(pyridin-3-yl)thiophene-2-carboxamide of Formula 1-75 was prepared by reacting 2-(4-chlorobutoxy)benzyl bromide with compound 7, with a yield of 48%. MS(ESI) (m/z): 417, 419[M+H]+. $^1H$ NMR (400 MHz, $CDCl_3$) δ 9.43 (s, 1H), 8.34 (s, 1H), 8.19 (s, 1H), 7.60 (s, 1H), 7.50 - 7.41 (m, 1H), 7.38 - 7.19 (m, 2H), 7.09 (d, J = 30.2 Hz, 2H), 5.49 (s, 2H), 4.08 (m, 2H), 3.58 (m, 2H), 1.90 (m, 4H).

Referring to the conditions and steps of the General Synthetic Method 2 in Example 3, 3-(2-(6-chlorohexyloxy)benzyloxy)-N-(pyridin-3-yl)thiophene-2-carboxamide of Formula 1-76 was prepared by reacting 2-(6-chlorohexyloxy)benzyl bromide with compound 7, with a yield of 47%. MS(ESI) (m/z): 445, 447[M+H]+. $^1H$ NMR (400 MHz, $CDCl_3$) δ 9.40 (d, J = 47.7 Hz, 1H), 8.27 (d, J = 57.3 Hz,3H), 7.60 (s, 1H), 7.32 (d, J = 16.6 Hz, 2H), 7.09 (d, J = 23.1 Hz, 3H), 5.46 (s, 2H), 4.04 (m, 2H), 3.46 (m, 2H), 1.73 (m, 4H), 1.42 (m, 4H).

Referring to the conditions and steps of the General Synthetic Method 2 in Example 3, 3-(thiophen-2-ylmethoxy)-N-(pyridin-3-yl)thiophene-2-carboxamide of Formula 1-77 was prepared by reacting 2-bromomethylthiophene with compound 7, with a yield of 9%. MS(ESI) (m/z): 317[M+H]+. $^1H$ NMR (400 MHz, $CDCl_3$) δ 9.18 (s, 1H), 8.32 (d, J = 19.4 Hz, 3H), 7.51 (d, J = 27.6 Hz, 2H), 7.26 - 7.16 (m, 2H), 7.06 (d, J = 26.8 Hz, 2H), 5.45 (s, 2H).

Referring to the conditions and steps of the General Synthetic Method 2 in Example 3, 3-(thiophen-3-ylmethoxy)-N-(pyridin-3-yl)thiophene-2-carboxamide of Formula 1-78 was prepared by reacting 3-bromomethylthiophene with compound 7, with a yield of 8%. MS(ESI) (m/z): 317[M+H]+. $^1H$ NMR (400 MHz, $CDCl_3$) δ 9.21 (s, 1H), 8.30 (m, 3H), 7.60 - 7.42 (m, 3H), 7.30 (m, 2H), 6.99 (s, 1H), 5.30 (s, 2H).

Referring to the conditions and steps of the General Synthetic Method 2 in Example 3, 3-(3-(hexyloxy)benzyloxy)-N-(pyridin-3-yl)thiophene-2-carboxamide of Formula 1-79 was prepared by reacting 3-(hexyloxy)benzyl bromide with compound 7, with a yield of 12%. MS(ESI) (m/z): 411[M+H]+. $^1H$ NMR (400 MHz, $CDCl_3$) δ 9.24 (s, 1H), 8.31 (m, 2H), 8.20 (s, 1H), 7.51 (s, 1H), 7.38 (s, 1H), 7.27 (s, 1H), 7.23 (s, 1H), 7.06 (s, 1H), 6.98 (d, J = 8.1 Hz, 3H), 5.24 (s, 2H), 3.95 (m, 2H), 1.76 (m, 2H), 1.43 (m, 2H), 1.32 (m, 4H), 0.89 (t, 3H).

Referring to the conditions and steps of the General Synthetic Method 2 in Example 3, 3-(3-chloro-2-(hexyloxy)benzyloxy)-N-(pyridin-3-yl)thiophene-2-carboxamide of Formula 1-80 was prepared by reacting 3-chloro-2-(hexyloxy) benzyl bromide with compound 7, with a yield of 54%. MS(ESI) (m/z): 445, 447[M+H]+. $^1$H NMR (400 MHz, $CDCl_3$) δ 9.20 (s, 1H), 8.41 (s, 1H), 8.27 (d, J = 28.6 Hz, 2H), 7.50 (d, J = 14.1 Hz, 2H), 7.37 (s, 1H), 7.16 (m, 2H), 7.02 (s, 1H), 5.31 (s, 2H), 4.04 (m, 2H), 1.75 (m, 2H), 1.38 (m, 2H), 1.22 (m, 4H), 0.82 (t, 3H).

Referring to the conditions and steps of the General Synthetic Method 2 in Example 3, 3-(2-ethylbenzyloxy)-N-(pyridin-3-yl)thiophene-2-carboxamide of Formula 1-81 was prepared by reacting 2-ethylbenzyl bromide with compound 7, with a yield of 82%. MS(ESI) (m/z): 339[M+H]+. $^1$H NMR (400 MHz, $CDCl_3$) δ 9.20 (s, 1H), 8.26 (d, J = 4.3 Hz, 1H), 8.17 (d, J = 7.8 Hz, 1H), 8.07 (s, 1H), 7.54 (d, J = 4.5 Hz, 1H), 7.44 (t, J = 7.8 Hz, 2H), 7.34 (dd, J = 16.6, 7.1 Hz, 2H), 7.24 - 7.18 (m, 1H), 7.04 (d, J = 4.4 Hz, 1H), 5.31 (s, 2H), 2.79 (q, J = 7.2 Hz, 2H), 1.26 (t, J = 7.2 Hz, 3H).

Referring to the conditions and steps of the General Synthetic Method 2 in Example 3, 3-(2-isopropoxybenzyloxy)-N-(pyridin-3-yl)thiophene-2-carboxamide of Formula 1-82 was prepared by reacting 2-isopropoxybenzyl bromide with compound 7, with a yield of 43%. MS(ESI) (m/z): 369[M+H]+. $^1$H NMR (400 MHz, $CDCl_3$) δ 9.37 (s, 1H), 8.37 - 8.17 (m, 3H), 7.52 (d, J = 5.5 Hz, 1H), 7.43 (dd, J = 15.9, 6.9 Hz, 2H), 7.23 (dd, J = 8.2, 4.9 Hz, 1H), 7.07 - 6.97 (m, 2H), 5.32 (s, 2H), 4.61 (dt, J = 12.0, 5.9 Hz, 1H), 1.27 (d, J = 6.0 Hz, 6H).

Referring to the conditions and steps of the General Synthetic Method 2 in Example 3, 2-[(2-(pyridin-3-ylcarbamoyl)thiophene-3-yl)oxymethyl]ethyl benzoate of Formula 1-84 was prepared by reacting ethyl 2-(bromomethyl)benzoate with compound 7, with a yield of 53%. MS(ESI) (m/z): 383[M+H]+. $^1$H NMR (400 MHz, $CDCl_3$) δ 9.94 (s, 1H), 8.24 (dd, J = 7.5, 1.3 Hz, 1H), 8.08 (dt, J = 7.5, 1.4 Hz, 1H), 8.02 (d, J = 1.3 Hz, 1H), 7.89 (d, J = 7.5 Hz, 1H), 7.77 (dd, J = 7.4, 1.4 Hz, 1H), 7.64 (td, J = 7.5, 1.4 Hz, 1H), 7.56 - 7.46 (m, 2H), 7.26 (t, J = 7.5 Hz, 1H), 6.55 (d, J = 7.5 Hz, 1H), 5.20 (s, 2H), 4.31 (q, J = 6.0 Hz, 2H), 1.38 (t, J = 6.0 Hz, 3H).

Referring to the conditions and steps of the General Synthetic Method 2 in Example 3, 3-(2-difluoro-methoxybenzyloxy)-N-(pyridin-3-yl)thiophene-2-carboxamide of Formula 1-85 was prepared by reacting 2-(difluoromethoxy) benzyl bromide with compound 7, with a yield of 47%. MS(ESI) (m/z): 377[M+H]+. $^1$H NMR (400 MHz, $CDCl_3$) δ 9.16 (s, 1H), 8.27 (m, 3H), 7.53 (d, J = 15.4 Hz, 3H), 7.34 (s, 1H), 7.25 - 7.16 (m, 2H), 7.01 (s, 1H), 6.60 (t, J = 73.2 Hz, 1H), 5.37 (s, 2H).

Referring to the conditions and steps of the General Synthetic Method 2 in Example 3, 3-(2-bromobenzyloxy)-N-(pyridin-3-yl)thiophene-2-carboxamide of Formula 1-86 was prepared by reacting 2-bromobenzyl bromide with compound 7, with a yield of 36%. MS(ESI) (m/z): 389, 391[M+H]+. $^1$H NMR (400 MHz, $CDCl_3$) δ 9.17 (s, 1H), 8.30 (m, 3H), 7.71 (d, J = 7.8 Hz, 1H), 7.52 (m, 2H), 7.42 (s, 1H), 7.33 (s, 2H), 7.01 (s, 1H), 5.38 (s, 2H).

Referring to the conditions and steps of the General Synthetic Method 2 in Example 3, 3-(yloxy)-N-(pyridin-3-yl) thiophene-2-carboxamide of Formula 1-87 was prepared by reacting 2-iodobenzyl bromide with compound 7, with a yield of 39%. MS(ESI) (m/z): 436,438[M+H]+. $^1$H NMR (400 MHz, $CDCl_3$) δ 9.14 (s, 1H), 8.28 (m, 3H), 7.98 (s, 1H), 7.57 - 7.41 (m, 3H), 7.25 - 7.21 (m, 1H), 7.17 (s, 1H), 7.00 (s, 1H), 5.32 (s, 2H).

Referring to the conditions and steps of the General Synthetic Method 2 in Example 3, 3-(5-fluoro-2-methylbenzyloxy)-N-(pyridin-3-yl)thiophene-2-carboxamide of Formula 1-88 was prepared by reacting 5-fluoro-2-methylbenzyl bromide with compound 7, with a yield of 38%. MS(ESI) (m/z): 343[M+H]+. $^1$H NMR (400 MHz, $CDCl_3$) δ 9.11 (s, 1H), 8.30 (s, 1H), 8.21 (m, 2H), 7.54 (s, 1H), 7.28 (s, 1H), 7.26 - 7.21 (m, 1H), 7.16 (d, J = 8.4 Hz, 1H), 7.07 (s, 1H), 6.99 (s, 1H), 5.26 (s, 2H), 2.40 (s, 3H).

Referring to the conditions and steps of the General Synthetic Method 2 in Example 3, 3-(2,5-dichlorobenzyloxy)-N-(pyridin-3-yl)thiophene-2-carboxamide of Formula 1-89 was prepared by reacting 2,5-dichlorobenzyl bromide with compound 7, with a yield of 48%. MS(ESI) (m/z): 379, 381[M+H]+. $^1$H NMR (400 MHz, $CDCl_3$) δ 9.07 (s, 1H), 8.37 (d, J = 33.1 Hz, 3H), 7.54 (m, 2H), 7.42 (d, J = 27.6 Hz, 2H), 6.98 (s, 1H), 5.37 (s, 2H).

Referring to the conditions and steps of the General Synthetic Method 2 in Example 3, 3-(2,5-dipropoxybenzyloxy)-N-(pyridin-3-yl)thiophene-2-carboxamide of Formula 1-93 was prepared by reacting 2,5-dipropoxybenzyl bromide with compound 7, with a yield of 90%. MS(ESI) (m/z): 427[M+H]+. $^1$H NMR (400 MHz, $CDCl_3$) δ 9.36 (s, 1H), 8.25 (d, J = 14.6 Hz, 3H), 7.53 (d, J = 29.3 Hz, 1H), 7.24 (s, 1H), 7.11 - 6.86 (m, 4H), 5.29 (s, 2H), 3.87 (m, 4H), 1.77 (m, 2H), 1.68 (m, 2H), 1.01 (t, 3H), 0.90 (t, 3H).

Referring to the conditions and steps of the General Synthetic Method 2 in Example 3, 3-[(1,1'-Biphenyl-2-yl) methoxy]-N-(pyridin-3-yl)thiophene-2-carboxamide of Formula 1-94 was prepared by reacting 2-bromo-2'-methylbiphenyl with compound 7, with a yield of 41%. MS(ESI) (m/z): 387[M+H]+. $^1$H NMR (400 MHz, DMSO-$d_6$) δ 9.29 (s, 1H), 8.45 (s, 1H), 8.28 (s, 1H), 7.96 (s, 1H), 7.79 (m, 2H), 7.49 (m, 2H), 7.39 (m, 7H), 6.97 (s, 1H), 5.36 (s, 2H).

Referring to the conditions and steps of the General Synthetic Method 2 in Example 3, 3-(3-trifluoromethylbenzyloxy)-N-(pyridin-3-yl)thiophene-2-carboxamide of Formula 1-97 was prepared by reacting 3-trifluoromethylbenzyl bromide with compound 7, with a yield of 43%. MS(ESI) (m/z): 379[M+H]+. $^1$H NMR (400 MHz, DMSO-$d_6$) δ 9.50 (s, 1H), 8.65 (s, 1H), 8.28 (s, 1H), 8.03 (m, 2H), 7.88 (d, J = 4.2 Hz, 2H), 7.75 (d, J = 7.1 Hz, 1H), 7.69 (d, J = 7.3 Hz, 1H), 7.37 (dd, J = 7.9, 4.4 Hz, 1H), 7.29 (d, J = 4.9 Hz, 1H), 5.52 (s, 2H).

Referring to the conditions and steps of the General Synthetic Method 2 in Example 3, 3-(2,5-dimethoxybenzyloxy)-N-(pyridin-3-yl)thiophene-2-carboxamide of Formula 1-98 was prepared by reacting 2,5-dimethoxybenzylbromide

with compound 7, with a yield of 52%. MS(ESI) (m/z): 371[M+H]+. $^1$H NMR (400 MHz, DMSO-$d_6$) δ 9.51 (s, 1H), 8.54 (s, 1H), 8.29 (s, 1H), 8.01 (d, J = 8.5 Hz, 1H), 7.87 (d, J = 5.5 Hz, 1H), 7.46 - 7.32 (m, 2H), 7.17 (s, 1H), 7.03 (d, J = 9.1 Hz, 1H), 6.96 (d, J = 8.9 Hz, 1H), 5.38 (s, 2H), 3.71 (s, 6H).

Referring to the conditions and steps of the General Synthetic Method 2 in Example 3, 3-(adamantan-1-ylmethoxy)-N-(pyridin-3-yl)thiophene-2-carboxamide of Formula 1-99 was prepared by reacting 1-(bromomethyl)adamantane with compound 7, with a yield of 98%. MS(ESI) (m/z): 369[M+H]+. $^1$H NMR (400 MHz, DMSO-$d_6$) δ 9.29 (s, 1H), 8.72 (s, 1H), 8.31 (s, 1H), 8.15 (s, 1H), 7.86 (d, J = 5.3 Hz, 1H), 7.52 - 7.38 (m, 1H), 7.21 (d, J = 5.4 Hz, 1H), 3.91 (s, 2H), 2.00 (m, 3H), 1.67 (m, 12H).

**Example 5: Preparation of 3-benzyloxy-N-(2-fluoropyridin-3-yl)thiophene-2-carboxamide of formula 1-90**

1) Synthesis of 3-benzyloxythiophene-2-benzoyl chloride

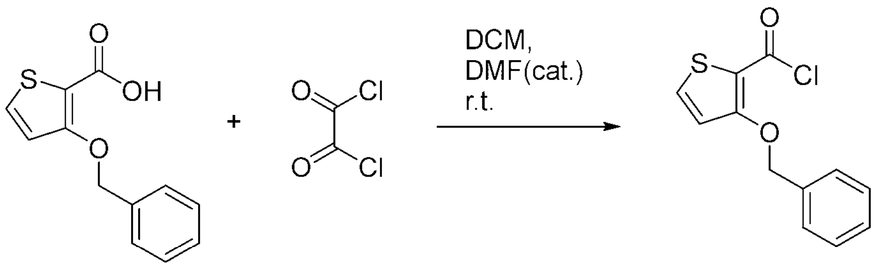


**5** **9**

1g (4.27mmol, 1.0eq) of compound 5 were weighed and placed into a round bottom flask, and 50 mL of DCM were added thereto for dissolution. After 5.4g (42.7mmol, 10.0eq) of oxalyl chloride were added while stirring in an ice bath, 500μl of DMF were added thereto for catalysis, and then the reaction solution was stirred for 7 hours at room temperature until the reaction was determined to be complete by TLC. After evaporating, EA was added, the reaction solution was then washed with saturated salt water once, dried with anhydrous sodium sulfate, and then be evaporated to obtain 996mg of pale-yellow solid, with a yield of 92%. The product can be used for the next reaction without further purification.

**2) Synthesis of 3-benzyloxy-N-(2-fluoropyridin-3-yl)thiophene-2-carboxamide of formula 1-90, General Synthetic Method 3**

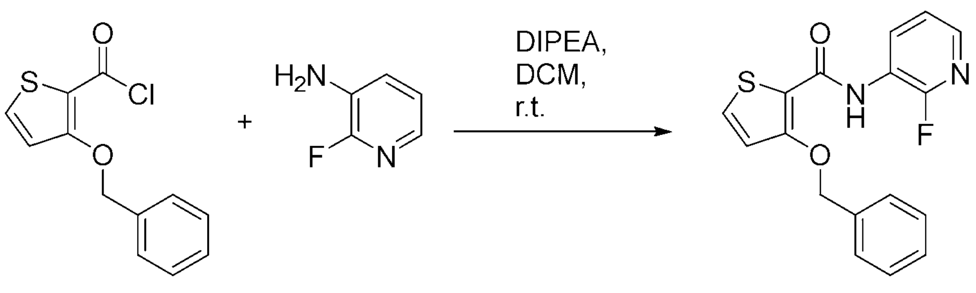


**9** **10** **1-90**

71mg (0.64mmol, 2.0eq) of compound 10 were weighed and placed into a round bottom flask, and 4 mL of DCM were added thereto for dissolution. After 82mg (0.64mmol, 2.0eq) of DIPEA were added, 80mg (0.32mmol, 1.0eq) of compound 9 were added while stirring, and then stirred overnight at room temperature until the reaction was determined to be complete by TLC. After the reaction was stopped, 100mg of white solid was obtained through flash purification, that is, 3-benzyloxy-N-(2-fluoropyridin-3-yl)thiophene-2-carboxamide of formula 1-90, with a yield of 96%. MS(ESI) (m/z): 329 [M+H]+. $^1$H NMR (400 MHz, $CDCl_3$) δ 9.45 (s, 1H), 8.89 (s, 1H), 7.82 (s, 1H), 7.53 - 7.39 (m, 5H), 7.26 - 7.23 (m, 1H), 7.16 (s, 1H), 6.98 (s, 1H), 5.31 (s, 2H).

**Example 6: Preparation of compounds 1-62, 1-91, and 1-92**

Referring to the conditions and steps of the General Synthetic Method 3 in Example 5, 3-benzyloxy-N-methyl-(pyridin-3-yl)thiophene-2-carboxamide of Formula 1-62 was prepared by reacting N-methyl-3-pyridinamine with compound 9, with a yield of 27%. MS(ESI) (m/z): 325[M+H]+. $^1$H NMR (400 MHz, $CDCl_3$) δ 8.35 (d, J = 7.5 Hz, 2H), 7.34 (t, J = 11.0 Hz, 4H), 7.23 (d, J = 5.4 Hz, 1H), 7.19 (d, J = 6.6 Hz, 2H), 7.07 (s, 1H), 6.50 (d, J = 4.2 Hz, 1H), 4.73 (s, 2H), 3.44 (s, 3H).

Referring to the conditions and steps of the General Synthetic Method 3 in Example 5, 3-benzyloxy-N-(4-chloropyridin-3-yl)thiophene-2-carboxamide of Formula 1-91 was prepared by reacting 3-amino-4-chloropyridine with compound 9, with a yield of 26%. MS(ESI) (m/z): 345,347 [M+H]+. $^1$H NMR (400 MHz, $CDCl_3$) δ 9.79 (s, 1H), 9.51 (s, 1H), 8.20 (s, 1H), 7.54 - 7.40 (m, 5H), 7.26 (s, 1H), 7.20 (s, 1H), 6.99 (s, 1H), 5.28 (s, 2H).

Referring to the conditions and steps of the General Synthetic Method 3 in Example 5, 3-benzyloxy-N-(5-fluoropyridin-3-yl)thiophene-2-carboxamide of Formula 1-92 was prepared by reacting 3-amino-5-fluoropyridine with compound 9, with a yield of 98%. MS(ESI) (m/z): 329[M+H]+. $^1$H NMR (400 MHz, $CDCl_3$) δ 9.36 (s, 1H), 8.22 (d, J = 9.7 Hz, 1H), 8.16 (s, 1H), 7.89 (s, 1H), 7.56 (d, J = 5.2 Hz,1H), 7.51 (m, 5H), 7.02 (d, J = 4.8 Hz, 1H), 5.30 (s, 2H).

**Example 7: Preparation of compounds 1-95 and 1-96**

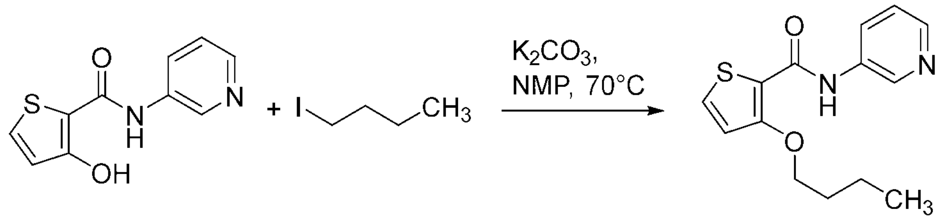


7 11 1-95

50mg (0.23mmol, 1.0eq) of compound 7 and 63mg (0.45mmol, 2.0eq) of potassium carbonate were weighed and placed into a round bottom flask, and 3 mL of NMP were added thereto for dissolution. After 50mg of compound 11 were added while stirring, the reaction solution was transferred into an oil bath and stirred overnight at 70 °C until the reaction was determined to be complete by TLC. After being dissolved by EA, washed with saturated salt water for three times, dried with anhydrous sodium sulfate and purified by flash, 75mg of yellow solid was obtained, with a yield of 48%. MS(ESI) (m/z): 277[M+H]+. $^1$H NMR (400 MHz, DMSO-$d_6$) δ 9.40 (s, 1H), 8.75 (s, 1H), 8.31 (s, 1H), 8.12 (s, 1H), 7.87 (s, 1H), 7.42 (s, 1H), 7.22 (s, 1H), 4.33 (m, 2H), 1.85 (m, 2H), 1.49 (m, 2H), 0.97 (t, 3H).

Referring to the preparation conditions and steps of the compound 1-95, 3-octyloxy-N-(pyridin-3-yl)thiophene-2-carboxamide of Formula 1-96 was prepared by reacting 8-iodooctane with compound 7, with a yield of 54%. MS(ESI) (m/z): 333[M+H]+. $^1$H NMR (400 MHz, DMSO-$d_6$) δ 9.39 (s, 1H), 8.74 (s, 1H), 8.30 (s, 1H), 8.10 (s, 1H), 7.86 (s, 1H), 7.39 (s, 1H), 7.21 (s, 1H), 4.30 (m, 2H), 1.84 (m, 2H), 1.49 - 1.18 (m, 10H), 0.80 (t, 3H).

**Example 8: Preparation of compounds 1-17 and 1-24**

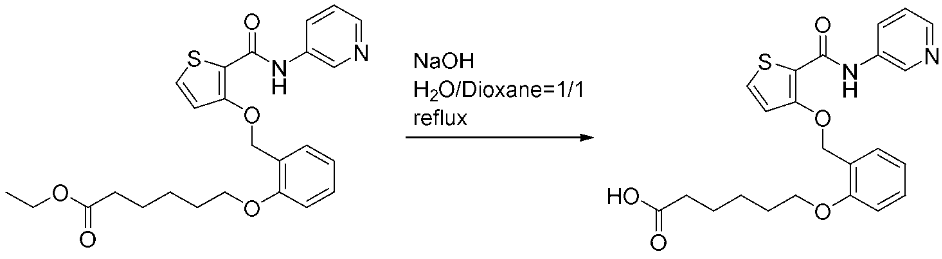


1-18 1-17

100mg of compound 1-18 were weighed and placed into a 100 mL round bottom flask, and 20 mL of hydroxide solution (2mol/L, H2O/dioxane=1/1) were added thereto at room temperature. The reaction solution was then transferred

into an oil bath for reflux for 8 hours until the reaction was determined to be complete by TLC. The pH of the reaction solution was adjusted to neutral with dilute hydrochloric acid after cooling. After being extracted by EA for three times, EA phases were combined, washed with saturated salt water once, dried with anhydrous sodium sulfate, and purified by flash, to obtain 80 mg of white solid, that is 6-(2-[(2-(pyridin-3-ylcarbamoyl)thiophene-3-yl)oxymethyl]phenoxy)hexanoic acid of Formula 1-17, with a yield of 85%. MS(ESI) (m/z): 441[M+H]+. $^1$H NMR (400 MHz, DMSO-$d_6$) δ 9.45 (s, 1H), 8.36 (s, 1H), 8.26 (d, J = 4.4 Hz, 1H), 7.95 (d, J = 7.5 Hz, 1H), 7.87 (d, J = 5.4 Hz, 1H), 7.53 (d, J = 7.3 Hz, 1H), 7.43 - 7.28 (m, 3H), 7.08 (d, J = 8.2 Hz, 1H), 7.00 (t, J = 7.3 Hz, 1H), 5.37 (s, 2H), 3.92 (t, J = 6.0 Hz, 2H), 2.07 (t, J = 7.3 Hz, 2H), 1.55 (d, J = 6.4 Hz, 2H), 1.37 (dd, J = 14.4, 7.2 Hz, 2H), 1.25 (d, J = 6.1 Hz, 2H).

Referring to the preparation conditions and steps of the compound 1-17, 3-(2-chloro-5-hydroxybenzyloxy)-N-(pyridin-3-yl)thiophene-2-carboxamide of Formula 1-24 was prepared by using the compound 1-23 as a reactant, with a yield of 69%. MS(ESI) (m/z): 361, 363[M+H]+. $^1$H NMR (400 MHz, DMSO-$d_6$) δ 9.88 (s, 1H), 9.43 (s, 1H), 8.59 (s, 1H), 8.29 (d, J = 4.4 Hz, 1H), 8.05 (d, J = 8.2 Hz, 1H), 7.89 (d, J = 5.4 Hz, 1H), 7.38 (dd, J = 8.0, 4.8 Hz, 1H), 7.33 (d, J = 8.6 Hz, 1H), 7.28 (d, J = 5.4 Hz, 1H), 7.08 (s, 1H), 6.81 (d, J = 8.6 Hz, 1H), 5.43 (s, 2H).

**Example 9: phosphate(4-chloro-3((2-(pyridin-3-ylcarbamoyl)thiophene-3-yl) oxymethyl))benzoate of Formula 1-26**

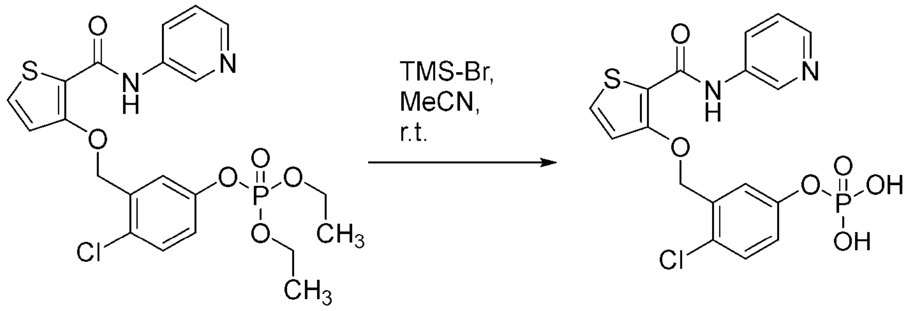


1-25 1-26

373mg (0.75mmol, 1.0eq) of compound 1-25 were weighed and placed into a round bottom flask, 12 mL of acetonitrile were added thereto for dissolution. After 1.15g (7.5mmol, 10.0eq) of TMS-Br were added while stirring, and the reaction solution was stirred for 48 hours at room temperature, meanwhile, a large amount of white solid was precipitated. After adding acetonitrile and ultrasonically filtering, 246 mg of white solid was obtained, that is phosphate(4-chloro-3((2-(pyridin-3-ylcarbamoyl)thiophene-3-yl) oxymethyl))benzoate of Formula 1-26, with a yield of 74%. MS(ESI) (m/z): 441, 443[M+H]+. $^1$H NMR (400 MHz, DMSO-$d_6$) δ 9.87 (s, 1H), 9.14 (s, 1H), 8.57 (s, 1H), 8.40 (d, J = 8.2 Hz, 1H), 7.95 (d, J = 5.3 Hz, 1H), 7.87 (d, J = 4.6 Hz, 1H), 7.53 (d, J = 8.6 Hz, 1H), 7.49 (s, 1H), 7.32 (d, J = 5.2 Hz, 1H), 7.24 (d, J = 8.5 Hz, 1H), 5.50 (s, 2H).

**Example 10: Preparation of hydrochloride salt of compound 1-2**

0.60 g (1.66 mmol, 1.0 eq) of compound 1-2 was dissolved in 10 mL of dry ethyl acetate, and then, 1.44 mL (1.8 mmol, 1.1 eq) of ethyl acetate solution (c = 1.25 mol/L) of HCl(g) was added dropwise to the reaction solution under an ice water bath condition. After 10 minutes of reaction, the reaction solution was vacuum filtered and dried to obtain 0.53 g of white powdery solid, with a yield of 81%.

**Example 11: Preparation of compound of formula 1-100**

Referring to the conditions and steps of the General Synthetic Method 1 in Example 1, 4-(benzyloxy)-N-(pyridin-3-yl)thiophene-3-carboxamide of formula 1-100 was synthesized via amide condensation between 4-(benzyloxy) thiophene-3-carboxylic acid and 3-Aminopyridine, with a yield of 80%.MS(ESI) (m/z): 311[M+H]+. $^1$H NMR (400 MHz, dmso) δ 9.89 (s, 1H), 8.63 (s, 1H), 8.27 (s, 1H), 8.17 (s, 1H), 8.07 (d, J = 8.0 Hz, 1H), 7.57 (d, J = 7.2 Hz, 2H), 7.46 - 7.33 (m, 4H), 6.94 (s, 1H), 5.21 (s, 2H).

**Comparative Example: Preparation of compounds 12-1 to 12-20**

Referring to the conditions and steps of the General Synthetic Method 1 in Example 1, 2-(2-ethylbenzyloxy)-N-(pyridin-3-yl)benzamide of Formula 12-1 was prepared by reacting 3-(2-ethylbenzyloxy)thiophene-2-carboxylic acid with compound 6, with a yield of 72%. MS(ESI) (m/z): 333[M+H]+. $^1$H NMR (400 MHz, DMSO-$d_6$) δ 11.06 (s, 1H), 9.18 (d, J = 1.6 Hz, 1H), 8.60 (d, J = 5.3 Hz, 1H), 8.44 (d, J = 8.5 Hz, 1H), 7.94 (dd, J = 8.5, 5.4 Hz, 1H), 7.64 (dd, J = 7.6, 1.6 Hz, 1H), 7.61-7.55 (m, 1H), 7.49 (d, J = 7.4 Hz, 1H), 7.40 (d, J = 8.3 Hz, 1H), 7.26 (dt, J = 13.9, 6.5 Hz, 2H), 7.19-7.10 (m, 2H), 5.27 (s, 2H), 2.68 (q, J = 7.5 Hz, 2H), 1.12 (t, J = 7.5 Hz, 3H).

Referring to the conditions and steps of the General Synthetic Method 1 in Example 1, 3-benzyloxy-N-(pyridazin-4-yl)thiophene-2-carboxamide of Formula 12-2 was prepared by reacting 4-aminopyridazine with compound 5, with a yield of 96%. MS(ESI) (m/z): 312[M+H]+. $^1$H NMR (400 MHz, DMSO-$d_6$) δ 9.74 (s, 1H), 9.19 (d, J = 1.8 Hz, 1H), 9.05 (d, J = 5.9 Hz, 1H), 7.97 (dd, J = 5.9, 2.7 Hz, 1H), 7.94 (d, J = 5.5 Hz, 1H), 7.60 (s, 1H), 7.59 (s, 1H), 7.41 (dt, J = 22.3, 7.2 Hz, 3H), 7.29 (d, J = 5.5 Hz, 1H), 5.46 (s, 2H).

Referring to the conditions and steps of the General Synthetic Method 1 in Example 1, 3-benzyloxy-N-(1,3,4-thiadiazol-2-yl)thiophene-2-carboxamide of Formula 12-3 was prepared by reacting 2-amino-1,3,4-thiadiazole with compound 5, with a yield of 33%. MS(ESI) (m/z): 318[M+H]+. $^1$H NMR (400 MHz, $CDCl_3$) δ 10.65 (s, 1H), 8.84 (s, 1H), 7.57 (s, 1H), 7.45 (m, 4H), 7.27 (s, 1H), 6.92 (s, 1H), 5.41 (s, 2H).

Referring to the conditions and steps of the General Synthetic Method 1 in Example 1, 3-benzyloxy-N-(4-fluoropyridin-2-yl)thiophene-2-carboxamide of Formula 12-4 was prepared by reacting 2-amino-4-fluoropyridine with compound 5, with a yield of 38%. MS(ESI) (m/z): 329[M+H]+. $^1$H NMR (400 MHz, $CDCl_3$) δ 9.89 (s, 1H), 8.23 (s, 1H), 8.13 (d, J = 9.1 Hz, 1H), 7.45 (d, J = 21.3 Hz, 3H), 7.27 (s, 1H), 6.89 (s, 1H), 6.77 (s, 1H), 5.39 (s, 2H).

Referring to the conditions and steps of the General Synthetic Method 2 in Example 3, 3-(2-(1,3-dioxoisoindolin-2-yl)benzyloxy)-N-(pyridin-3-yl)thiophene-2-carboxamide of Formula 12-5 was prepared by reacting 2-(1,3-dioxoisoindolin-2-yl)benzyl bromide with compound 7, with a yield of 50%. MS(ESI) (m/z): 456[M+H]+. $^1$H NMR (400 MHz, $CDCl_3$) δ 9.97 (s, 1H), 8.44 (m, 1H), 8.34 (m, 1H), 8.13 (s, 1H), 7.87 (m, 1H), 7.79 - 7.72 (m, 4H), 7.61 (s, 1H), 7.49 - 7.37 (m , 2H), 7.32 (t, 1H), 7.24 - 7.19 (m, 1H), 6.63 (s, 1H), 5.24 (s, 2H).

Referring to the conditions and steps of the General Synthetic Method 1 in Example 1, 3-(benzyloxy)-N(dibenzo [b,d]furan-3-yl)thiophene-2-carboxamide of Formula 12-6 was prepared by reacting 3-aminedibenzo[b,d]furan with compound 5, with a yield of 75%. MS(ESI) (m/z): 400[M+H]+. $^1$H NMR (400 MHz, DMSO-$d_6$) δ 9.65 (s, 1H), 8.12 (s, 1H), 8.06 (t, J = 6.9 Hz, 2H), 7.88 (d, J = 5.4 Hz, 1H), 7.66 (dd, J = 17.7, 7.5 Hz, 3H), 7.51 - 7.35 (m, 5H), 7.31 (d, J = 5.2 Hz, 1H), 7.24 (d, J = 8.2 Hz, 1H), 5.46 (s, 2H).

Referring to the conditions and steps of the General Synthetic Method 1 in Example 1, 3-(benzyloxy)-N-(9H-fluoren-2-yl)thiophene-2-carboxamide of Formula 12-7 was prepared by reacting 2-amino-9-hydroxymethylfluorene with compound 5, with a yield of 38%. MS(ESI) (m/z): 398[M+H]+. $^1$H NMR (400 MHz, DMSO-$d_6$) δ 9.48 (s, 1H), 7.83 (dd, J = 11.6, 6.7 Hz, 3H), 7.77 (s, 1H), 7.62 (d, J = 7.0 Hz, 2H), 7.56 (d, J = 7.0 Hz, 1H), 7.52 - 7.39 (m, 4H), 7.39 - 7.23 (m, 3H), 5.45 (s, 2H), 3.90 (s, 2H).

Referring to the conditions and steps of the General Synthetic Method 2 in Example 3, 3-(2-(2-((5-methylpyridin-2-yl)amino)-2-oxoethyl)benzyloxy)-N-(pyridin-3-yl) thiophene-2-carboxamide of Formula 12-8 was prepared by reacting 2-(2-((5-methylpyridin-2-yl)amino)-2-oxoethyl)benzyl bromide with compound 7, with a yield of 63%. MS(ESI) (m/z): 459[M+H]+. $^1$H NMR (400 MHz, DMSO-$d_6$) δ 10.66 (s, 1H), 9.26 (s, 1H), 8.42 (s, 1H), 8.24 (s, 1H), 8.05 (s, 1H), 7.93 (d, J = 8.3 Hz, 1H), 7.84 (s, 1H), 7.74 (d, J = 8.7 Hz, 1H), 7.58 (s, 1H), 7.47 - 7.38 (m, 3H), 7.31 (dd, J = 21.7, 5.8 Hz, 3H), 5.48 (s, 2H), 3.95 (s, 2H), 2.19 (s, 3H).

Referring to the conditions and steps of the General Synthetic Method 1 in Example 1, 4-(benzyloxy)-2-methyl-N-(pyridin-3-yl)thiazole-5-carboxamide of Formula 12-9 was prepared by reacting 4-(benzyloxy)-2-methyl-N-(pyridin-3-yl)thiazole-5-carboxylic acid with compound 6, with a yield of 77%. MS(ESI) (m/z): 326[M+H]+. $^1$H NMR (400 MHz, DMSO-$d_6$) δ 9.19 (s, 1H), 8.46 (s, 1H), 8.12 (s, 1H), 7.85 (d, J = 8.0 Hz, 1H), 7.41 (d, J = 6.9 Hz, 2H), 7.23 (dd, J = 14.6, 6.8 Hz, 4H), 5.41 (s, 2H), 2.50 (s, 3H).

Referring to the conditions and steps of the General Synthetic Method 1 in Example 1, 3-(benzyloxy)-N-(3,4,5-trifluorophenyl)thiazole-2-carboxamide of Formula 12-10 was prepared by reacting 3,4,5-trifluoroaniline with compound 5, with a yield of 50%. MS(ESI) (m/z): 364[M+H]+. $^1$H NMR (400 MHz, $CDCl_3$) δ 9.24 (s, 1H), 7.55 (m, 5H), 7.32 (s, 1H), 7.06 (d, J = 21.9 Hz, 3H), 5.44 (s, 2H).

Referring to the conditions and steps of the General Synthetic Method 1 in Example 1, 3-(benzyloxy)-N-(pyridin-4-yl)thiazole-2-carboxamide of Formula 12-11 was prepared by reacting 4-aminopyridine with compound 5, with a yield of 97%. MS(ESI) (m/z): 311[M+H]+. $^1$H NMR (400 MHz, $CDCl_3$) δ 9.30 (s, 1H), 8.41 (m, 2H), 7.50 (m, 5H), 7.26 (m, 3H), 6.99 (s, 1H), 5.28 (s, 2H).

Referring to the conditions and steps of the General Synthetic Method 1 in Example 1, 3-(benzyloxy)-N-(6-methylpyridin-3-yl)thiazole-2-carboxamide of Formula 12-12 was prepared by reacting 2-amino-6-methylpyridine with compound 5, with a yield of 94%. MS(ESI) (m/z): 325[M+H]+. $^1$H NMR (400 MHz, DMSO-$d_6$) δ 9.38 (s, 1H), 8.43 (s, 1H),

7.91 (d, J = 8.1 Hz, 1H), 7.85 (s, 1H), 7.58 (m, 2H), 7.44 (s, 3H), 7.28 (s, 1H), 7.22 (d, J = 8.7 Hz, 1H), 5.42 (s, 2H), 2.41 (s, 3H).

Referring to the conditions and steps of the General Synthetic Method 1 in Example 1, 3-(benzyloxy)-N-(6-fluoropyridin-3-yl)thiazole-2-carboxamide of Formula 12-13was prepared by reacting 3-amino-6-fluoropyridine with compound 5, with a yield of 20%. MS(ESI) (m/z): 329[M+H]+. $^1$H NMR (400 MHz, $CDCl_3$) δ 9.23 (s, 1H), 8.26 (s, 1H), 7.85 (s, 1H), 7.51 (d, J = 19.4 Hz, 5H), 7.27 (s, 1H), 7.00 (s, 1H), 6.88 (s, 1H), 5.28 (s, 2H).

Referring to the conditions and steps of the General Synthetic Method 1 in Example 1, 3-(benzyloxy)-N-(6-trifluoromethylpyridin-3-yl)thiazole-2-carboxamide of Formula 12-14 was prepared by reacting 3-amino-6-(trifluoromethyl)pyridine with compound 5, with a yield of 48%. MS(ESI) (m/z): 379[M+H]+. $^1$H NMR (400 MHz, DMSO-$d_6$) δ 9.76 (s, 1H), 8.73 (s, 1H), 8.34 (d, J = 7.4 Hz, 1H), 7.90 (m, 2H), 7.59 (d, J = 6.4 Hz, 2H), 7.47 - 7.37 (m, 3H), 7.28 (d, J = 5.2 Hz, 1H), 5.45 (s, 2H).

Referring to the conditions and steps of the General Synthetic Method 1 in Example 1, 3-benzyloxy-N-(pyridin-3-yl)pyridin-2-carboxamide of Formula 12-15 was prepared by reacting 3-(benzyloxy)pyridin-2-carboxylic acid with compound 6, with a yield of 43%. MS(ESI) (m/z): 306[M+H]+. $^1$H NMR (400 MHz, DMSO-$d_6$) δ 10.67 (s, 1H), 8.87 (d, J = 2.5 Hz, 1H), 8.28 (dd, J = 4.7, 1.5 Hz, 1H), 8.26 - 8.21 (m, 1H), 8.18 (d, J = 8.1 Hz, 1H), 7.75 - 7.67 (m, 1H), 7.54 (dd, J = 8.5, 4.6 Hz, 1H), 7.46 (d, J = 7.2 Hz, 2H), 7.41 - 7.31 (m, 3H), 7.29 (d, J = 7.2 Hz, 1H), 5.25 (s, 2H).

Referring to the conditions and steps of the General Synthetic Method 1 in Example 1, 4-benzyloxy-N-(pyridin-3-yl)pyridin-3-carboxamide of Formula 12-16 was prepared by reacting 4-(benzyloxy)pyridin-3-carboxylic acid with compound 6, with a yield of 30%. MS(ESI) (m/z): 306[M+H]+. $^1$H NMR (400 MHz, DMSO-$d_6$) δ 10.33 (s, 1H), 8.61 (d, J = 2.4 Hz, 1H), 8.53 (s, 1H), 8.45 (d, J = 5.8 Hz, 1H), 8.16 (d, J = 4.8 Hz, 1H), 7.99 (d, J = 8.4 Hz, 1H), 7.39 (d, J = 7.3 Hz, 1H), 7.31 (t, J = 6.0 Hz, 1H), 7.26 - 7.18 (m, 4H), 7.07 - 7.01 (m, 1H), 5.19 (s, 2H).

Referring to the conditions and steps of the General Synthetic Method 1 in Example 1, 3-benzyloxy-N-(pyridin-3-yl)pyridin-4-carboxamide of Formula 12-17 was prepared by reacting 3-(benzyloxy)pyridin-4-carboxylic acid with compound 6, with a yield of 40%. MS(ESI) (m/z): 306[M+H]+. $^1$H NMR (400 MHz, DMSO-$d_6$) δ 10.58 (s, 1H), 8.69 (d, J = 34.6 Hz, 2H), 8.32 (dd, J = 22.3, 4.6 Hz, 2H), 8.09 (d, J = 8.2 Hz, 1H), 7.52 (dd, J = 28.8, 5.9 Hz, 3H), 7.35 (dd, J = 16.5, 9.5 Hz, 4H), 5.34 (s, 2H).

Referring to the conditions and steps of the General Synthetic Method 1 in Example 1, 2-benzyloxy-N-(pyridin-3-yl)pyridin-3-carboxamide of Formula 12-18 was prepared by reacting 2-(benzyloxy)pyridin-3-carboxylic acid with compound 6, with a yield of 36%. MS(ESI) (m/z): 306[M+H]+. $^1$H NMR (400 MHz, DMSO-$d_6$) δ 10.46 (s, 1H), 8.74 (d, J = 2.6 Hz, 1H), 8.42 - 8.34 (m, 1H), 8.31 (d, J = 4.7 Hz, 1H), 8.12 (d, J = 7.3 Hz, 2H), 7.54 (d, J = 7.2 Hz, 2H), 7.43 - 7.28 (m, 4H), 7.21 (dd, J = 7.3, 4.9 Hz, 1H), 5.49 (s, 2H).

Referring to the conditions and steps of the General Synthetic Method 1 in Example 1, 4-benzyloxy-N-(pyridin-3-yl)pyrimidin-5-carboxamide of Formula 12-19 was prepared by reacting 4-(benzyloxy)pyrimidin-5-carboxylic acid with compound 6, with a yield of 60%. MS(ESI) (m/z): 307[M+H]+. $^1$H NMR (400 MHz, DMSO-$d_6$) δ 10.56 (s, 1H), 8.95 (s, 1H), 8.85 (s, 1H), 8.75 (d, J = 2.5 Hz, 1H), 8.30 (dd, J = 4.7, 1.5 Hz, 1H), 8.13 - 8.06 (m, 1H), 7.55 - 7.45 (m, 2H), 7.41 - 7.30 (m, 4H), 5.53 (s, 2H).

Referring to the conditions and steps of the General Synthetic Method 1 in Example 1, 3-benzyloxy-N-(pyridin-3-yl)piperazin-2-carboxamide of Formula 12-20 was prepared by reacting 3-(benzyloxy)piperazin-2-carboxylic acid with compound 6, with a yield of 60%. MS(ESI) (m/z): 307[M+H]+. $^1$H NMR (400 MHz, DMSO-$d_6$) δ 10.73 (s, 1H), 8.73 (d, J = 2.5 Hz, 1H), 8.31 (d, J = 2.6 Hz, 1H), 8.22 (d, J = 2.6 Hz, 1H), 8.18 (dd, J = 4.7, 1.4 Hz, 1H), 8.04 (dd, J = 8.5, 1.9 Hz, 1H), 7.34 (d, J = 7.2 Hz, 2H), 7.30 - 7.25 (m, 1H), 7.25 - 7.20 (m, 2H), 7.19 - 7.14 (m, 1H), 5.35 (s, 2H).

The structures of the comparative compounds are as follows:

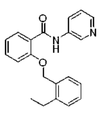
12-1

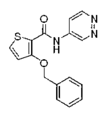
12-2

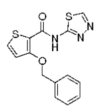
12-3

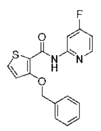
12-4

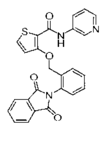
12-5

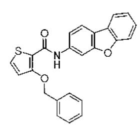
12-6

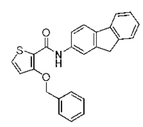
12-7

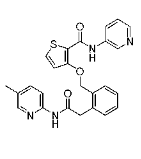
12-8

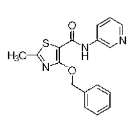
12-9

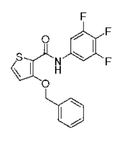
12-10

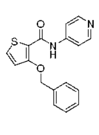
12-11

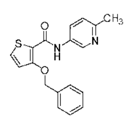
12-12

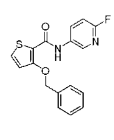
12-13

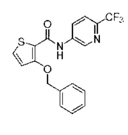
12-14

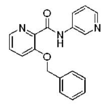
12-15

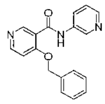
12-16

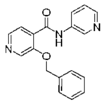
12-17

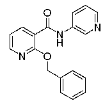
12-18

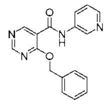
12-19

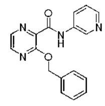
12-20

Example 12: Determination of the SMS2 inhibitory activity in vitro of the alkoxy thiophene acyl arylamine compounds, including a prodrug which is a carboxyl/phosphate **compound derived from the alkoxy thiophene acyl arylamine compound**

Experimental instruments and materials

1. Electro-thermostatic water bath, from Shanghai Yiheng Technology Co., Ltd.);
2. Vortex Mixer, with model ID of XW-80A, from Shanghai Jingke Industrial Co., Ltd.;
3. High speed centrifuge, with model ID of Eppendorf 5804R;
4. High Performance Liquid Chromatography Agilent 1100, equipped with a quaternary pump, vacuum degassing, and FLD fluorescence detector, from Agilent Technologies, Palo Alto, CA, USA;
5. HPLC column COSMOSIL 5C18-MS-II (4.6mm I.D. X 250mm);
6. DMPC, purchased from Santa Cruz (USA), dissolved with ethanol, with a concentration of 40 mmol/L. C6-NBD-Ceramide, purchased from Santa Cruz (USA), dissolved with DMSO, with a concentration of 1.16 mmol/L;
7. The organic solvents used in the present disclosure were purchased from Shanghai National Reagent Company, wherein methanol was pure chromatography; water was ultra-pure water which is filtrated by Milli-Q pump, deionized and ultra-filtrated with 0.22 μm membrane; and other biological consumables were purchased from domestic companies;
8. Preparation of test compound solutions: each test compound was accurately weighed and dissolved in an appropriate volume of DMSO to prepare a 10 mmol/L stock solution. The stock solution was then diluted with an appropriate volume of DMSO to achieve the desired working concentration;
9. SMS2 pure enzyme, DDM solution and buffer solution were provided by CAO, Yu research team from National Protein Scientific Center (Shanghai).

Determination of the SMS2 inhibitory activity of the alkoxy thiophene acyl arylamine compounds, including a prodrug which is a carboxyl/phosphate compound derived from the alkoxy thiophene acyl arylamine compound

6 μl (0.144 mg/mL) of SMS2 protein were diluted in 3 mL of DDM buffer solution with vortex mixing. 79 μl of the diluted enzyme solution were transferred into a 1.5 mL EP tube, 1 μl of inhibitor DMSO solution were added thereto, vortexing for 15 seconds, followed by standing at room temperature for 5 minutes. 20 μl of a mixed solution of DMPC and C6-NBD-ceramide were added, vortexing for 15 seconds, and incubated at 37°C for 30 minutes. 200 μl of anhydrous ethanol were added to quench the reaction, vortexing for 15 seconds. 200 μl of the reaction solution were taken out and stored at 4°C and avoided light for HPLC detection.

Referring to the literature (Xiaodong Deng, et al. Analytical Letters, 2012, 45(12): 1581-1589.), the fluorescence quantitative analysis of the prepared samples was conducted using the same high-performance liquid chromatography method as in the literature. The peak area values of A(sm) and A(ceramide) in the HPLC chromatogram corresponding to C6-NBD-SM and C6-NBD-Ceramide in the DMSO group and inhibitor group samples were analyzed and recorded, and the inhibition rate of the test compound was calculated according to the following formula.

$$\text{Inhibition rate} = \frac{\textit{Peak area values of DMSO group} - \textit{Peak area values of Inhibitor group}}{\textit{Peak area values of DMSO group}} \times 100\%$$

The inhibition rates of the target compounds, including the comparative compounds, on SMS2 at concentrations of 1 μM or 10 μM were measured as follows:

| ID | C/μM 1 | C/μM 10 | $IC_{50}$ (nM) |
|---|---|---|---|
| 1- 1 | 56 | - | - |
| 1- 2 | 81 | - | 69 |
| 1- 3 | - | 97 | - |
| 1- 4 | - | 63 | - |
| 1- 5 | 47 | - | - |
| 1- 6 | - | 50 | - |
| 1- 7 | - | 50 | - |
| 1- 8 | - | 56 | - |
| 1- 9 | - | 50 | - |
| 1- 10 | 86 | - | - |
| 1- 11 | - | 50 | - |
| 1- 12 | 68 | - | - |
| 1- 13 | 50 | - | - |
| 1- 14 | - | 50 | - |
| 1- 15 | - | 53 | - |
| 1- 16 | 78 | - | - |
| 1- 17 | - | 50 | - |
| 1- 18 | - | 97 | - |
| 1- 19 | - | 77 | - |
| 1- 20 | 50 | - | - |
| 1- 21 | - | 93 | - |
| 1- 22 | - | 81 | - |
| 1- 23 | - | 53 | - |
| 1- 24 | 78 | - | - |
| 1- 25 | 1 | - | - |

| ID | C/μM 1 | C/μM 10 |
|---|---|---|
| 1- 26 | 13 | - |
| 1- 27 | 96 | - |
| 1- 28 | 97 | - |
| 1- 29 | 95 | - |
| 1- 30 | 95 | - |
| 1- 31 | 96 | - |
| 1- 32 | 97 | - |
| 1- 33 | 94 | - |
| 1- 34 | 95 | - |
| 1- 35 | 91 | - |
| 1- 36 | 85 | - |
| 1- 37 | 89 | - |
| 1- 38 | 91 | - |
| 1- 39 | 74 | - |
| 1- 40 | 23 | - |
| 1- 41 | 72 | - |
| 1- 42 | - | 50 |
| 1- 43 | 69 | - |
| 1- 44 | 54 | - |
| 1- 45 | 67 | - |
| 1- 46 | - | 70 |
| 1- 47 | 74 | - |
| 1- 48 | 74 | - |
| 1- 49 | - | 51 |
| 1- 50 | - | 66 |

| ID | C/μM 1 | C/μM 10 |
|---|---|---|
| 1- 51 | - | 60 |
| 1- 52 | - | 50 |
| 1- 53 | 87 | - |
| 1- 54 | - | 57 |
| 1- 55 | - | 65 |
| 1- 56 | - | 50 |
| 1- 57 | 87 | - |
| 1- 58 | 70 | - |
| 1- 59 | 71 | - |
| 1- 60 | 78 | - |
| 1- 61 | - | 50 |
| 1- 62 | - | 57 |
| 1- 63 | 80 | - |
| 1- 64 | - | 51 |
| 1- 65 | - | 61 |
| 1- 66 | 86 | - |
| 1- 67 | 67 | - |
| 1- 68 | - | 50 |
| 1- 69 | - | 65 |
| 1- 70 | 95 | - |
| 1- 71 | 95 | - |
| 1- 72 | 90 | - |
| 1- 73 | 90 | - |
| 1- 74 | 98 | - |
| 1- 75 | 97 | - |

| ID | C/μM 1 | C/μM 10 |
|---|---|---|
| 1- 76 | 93 | - |
| 1- 77 | - | 65 |
| 1- 78 | - | 59 |
| 1- 79 | - | 60 |
| 1- 80 | 97 | - |
| 1- 81 | 73 | - |
| 1- 82 | 80 | - |
| 1- 83 | - | 50 |
| 1- 84 | 63 | - |
| 1- 85 | 73 | - |
| 1- 86 | 86 | - |
| 1- 87 | 82 | - |
| 1- 88 | 76 | - |
| 1- 89 | 74 | - |
| 1- 90 | 56 | - |
| 1- 91 | - | 56 |
| 1- 92 | 50 | - |
| 1- 93 | - | 50 |
| 1- 94 | 52 | - |
| 1- 95 | - | 50 |
| 1- 96 | - | 50 |
| 1- 97 | 80 | - |
| 1- 98 | 50 | - |
| 1- 99 | - | 62 |
| 1- 100 | 60 | - |

| ID | C/μM 1 | C/μM 10 | $IC_{50}$ (nM) |
|---|---|---|---|
| 12-1 (LY93) | 66 | - | 285 |
| 12-2 | - | 23 | - |
| 12-3 | -8 | - | - |
| 12-4 | 3 | - | - |
| 12-5 | - | 4 | - |
| 12-6 | -5 | - | - |
| 12-7 | -16 | - | - |
| 12-8 | - | 7 | - |
| 12-9 | -2 | - | - |
| 12-10 | -30 | - | - |
| 12-11 | 7 | - | - |
| 12-12 | 7 | - | - |
| 12-13 | 9 | - | - |
| 12-14 | - | 10 | - |
| 12-15 | - | 16 | - |
| 12-16 | - | 17 | - |
| 12-17 | - | 27 | - |
| 12-18 | - | 22 | - |
| 12-19 | - | 18 | - |
| 12-20 | - | 16 | - |

(Each set of data was measured twice and the average value was taken; "-" indicates that it was not measured.)

**Example 13: Evaluation of Therapeutic Effect of SMS2 Inhibitor on Dry Eye of Mice**

(1) Experimental Materials

Experimental animals: male C57BL/6 experimental mice aged 6-8 weeks.

Experimental instrument: an Alisn thermostatic blast drying oven, a desktop microscope, and a cobalt blue lamp.

Experimental reagents and consumables: phenol red cotton line, 1% sodium fluorescein solution, 兹润® 0.05% cyclosporin eyedrops (II) from Xingqi Eyedrops Company, 0. 04 mg/g of compound 1-2 formulation, 0.04 mg/g of compound 1-31 formulation.

(2) Experimental Method

28 male C57BL/6 experimental mice aged 6-8 weeks were placed in a thermostatic blast drying oven with controlled environmental humidity of 19.5% and temperature of 25°C, and were kept for 2 weeks to establish dry eye models. At the end of 2 weeks, the dry eye mice were randomly divided into four groups, with 7 mice in each group.

Subsequently, the humidity of the thermostatic blast drying oven was adjusted to 50% and the mice were continuously fed for one week, meanwhile each group of mice received a corresponding drug treatment: mice in a carrier group were treated with an empty carrier containing no SMS2 inhibitor; mice in a cyclosporine group was treated by using

commercial cyclosporine eyedrops; mice in the group of the compound 1-2 were treated with 0.04% compound 1-2; and, mice in the group of the compound 1-31 were treated with 0.04% compound 1-31. Each eye of every mouse received 5 μl of pharmaceutical industry through ocular surface administration, twice daily. Another group of 7 male C57BL/6 experimental mice aged 6-8 weeks was kept in a thermostatic blast drying oven with controlled environmental humidity of 50% and temperature of 25°C, and received an empty carrier as a physiological control in the third week.

All mice were evaluated for ocular surface damage recovery by ocular surface fluorescein sodium staining scores on the third weekend, and the tear secretion of mice was measured with phenol red surface lines. To be more specific, the phenol red surface line was placed in the conjunctival sac of a mouse in the anesthesia state for 30 seconds, and then the discoloration length of the phenol red surface line was measured. 2 μl of 1% sodium fluorescein solution was then administered through ocular surface, and photographs were taken under a cobalt blue lamp for scoring and statistics.

Experimental results demonstrate that the SMS2 inhibitors of the present disclosure have a significant therapeutic effect on treating corneal epithelial damage caused by dry eye, and simultaneously promotes tear secretion in dry eye mice. The two SMS2 inhibitors, that is compound 1-2 and compounds 1-31 in examples of the present disclosure, all demonstrated efficacy in the treatment of dry eye (as shown in Figure 1 and Figure 2), manifested as a reduction in corneal fluorescein sodium staining scores, and exhibited statistically significant advantages compared to cyclosporine eyedrops, a commonly used commercial preparation for dry eye treatment. In terms of promoting tear secretion (as shown in Figure 3), cyclosporine eyedrops and both the SMS2 inhibitors of the present disclosure exhibited an increasing trend compared to the vehicle group. These experimental results demonstrate that SMS2 inhibitors of the present disclosure have potential for the treatment of dry eye.

The above examples are only preferred embodiments of the present disclosure. It should be noted that one skilled in the relevant art can also make several improvements and modifications without departing from the principles of the present disclosure, and these improvements and modifications should also be regarded as the protection scope of the present disclosure.

## Claims

1. An alkoxy thiophene acyl arylamine compound of Formula (I), or a pharmaceutically acceptable salt thereof,

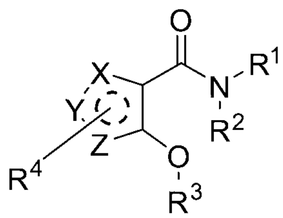

(I)

wherein X, Y and Z are each independently selected from the group consisting of carbon atom and sulfur atom;
$R^1$ is phenyl, pyridyl, pyridazinyl, pyrimidinyl or thiazolyl, wherein such groups are unsubstituted or substituted with 1, 2, 3, 4 or 5 substituents independently selected from the group consisting of halogen, methyl, hydroxyl, methoxy, trifluoromethyl, trifluoromethoxy, cyano, and nitro;
$R^2$ is hydrogen, an alkyl group having 1 to 3 carbon atoms, or cycloalkyl;
$R^3$ is a substituted or unsubstituted hydrocarbyl group;
$R^4$ is one or two groups selected from the group consisting of hydrogen, alkyl group having 1 to 3 carbon atoms, halogen, and nitro.

2. The alkoxy thiophene acyl arylamine compound or a pharmaceutically acceptable salt thereof according to claim 1, **characterized in that** X and Y are each independently selected from the group consisting of carbon atom and sulfur atom, and Z is carbon atom.

3. The alkoxy thiophene acyl arylamine compound or a pharmaceutically acceptable salt thereof according to claim 1, **characterized in that** $R^3$ is a group having one of the following structures:

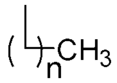 , 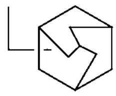 , 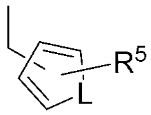 , 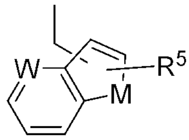 , 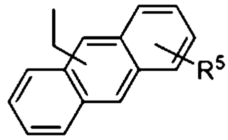 ,

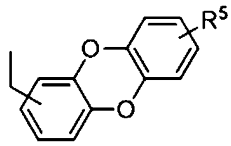 ,

wherein n is an integer from 1 to 10;
W is carbon atom or nitrogen atom;
L, M are each independently selected from the group consisting of -C=C-, sulfur atom and oxygen atom;
$R^5$ is 1 to 5 groups selected from the group consisting of hydrogen, a substituted or unsubstituted alkyl group having 1 to 4 carbon atoms, a substituted or unsubstituted alkoxy group having 1 to 20 carbon atoms, halogen, acyl group, nitro, cyano, hydroxyl, carboxylate group, phosphate group and heteroaromatic ring.

**4.** The alkoxy thiophene acyl arylamine compound or a pharmaceutically acceptable salt thereof according to claim 3, **characterized in that** the substituted or unsubstituted alkoxy group has 1 to 15 carbon atoms.

**5.** The alkoxy thiophene acyl arylamine compound or a pharmaceutically acceptable salt thereof according to claim 1, **characterized in that** the alkoxy thiophene acyl arylamine compound is:

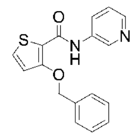
1-1

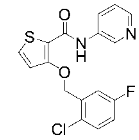
1-2

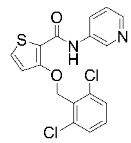
1-3

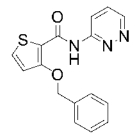
1-4

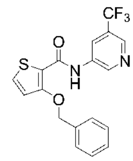
1-5

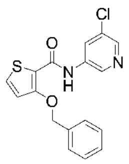
1-6

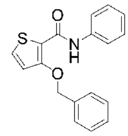
1-7

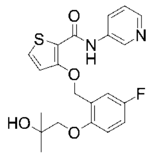
1-8

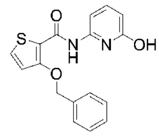
1-9

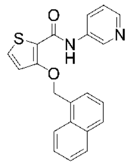
1-10

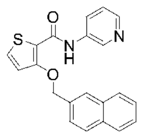
1-11

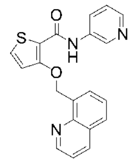
1-12

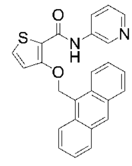
1-13

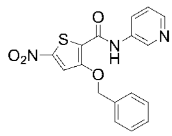
1-14

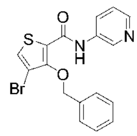
1-15

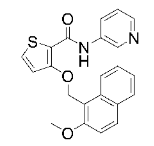
1-16

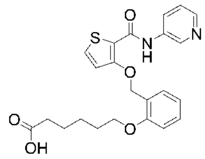
1-17

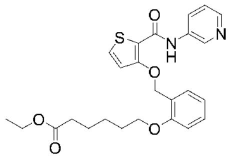
1-18

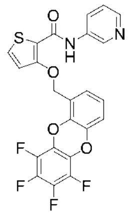
1-19

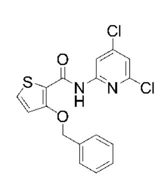
1-20

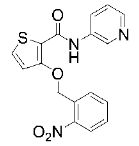
1-21

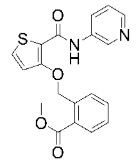
1-22

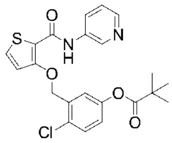
1-23

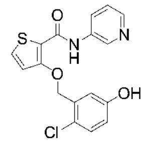
1-24

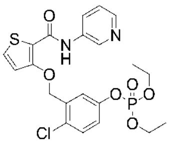
1-25

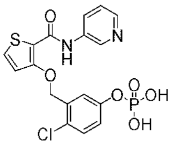
1-26

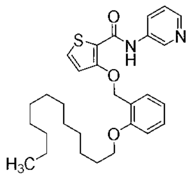
1-27

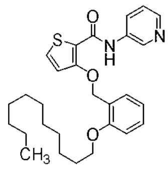
1-28

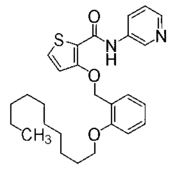
1-29

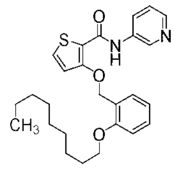
1-30

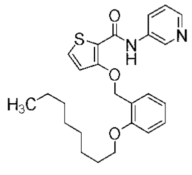
1-31

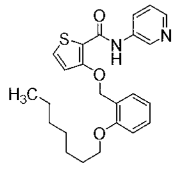
1-32

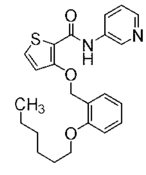
1-33

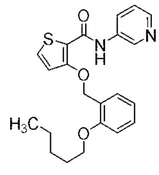
1-34

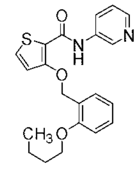
1-35

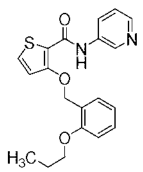
1-36

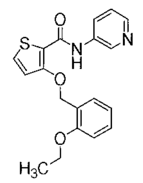
1-37

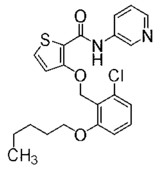
1-38

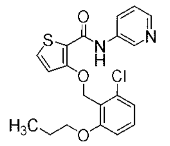
1-39

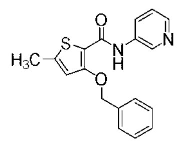
1-40

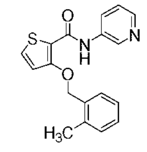
1-41

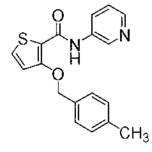
1-42

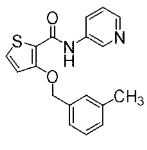
1-43

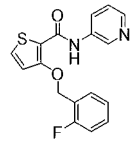
1-44

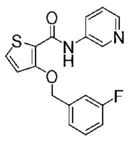
1-45

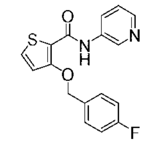
1-46

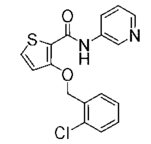
1-47

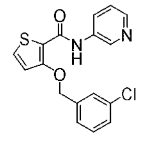
1-48

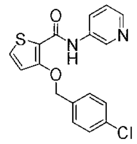
1-49

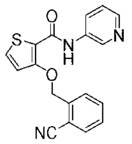
1-50

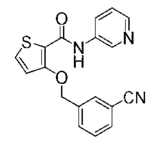
1-51

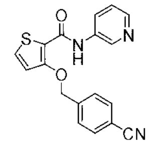
1-52

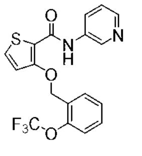
1-53

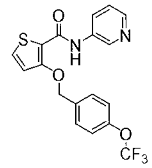
1-54

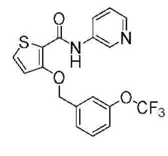
1-55

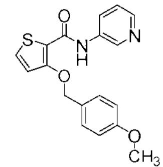
1-56

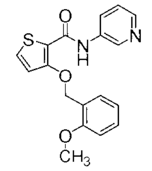
1-57

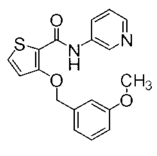
1-58

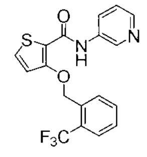
1-59

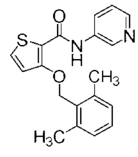
1-60

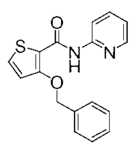
1-61

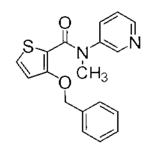
1-62

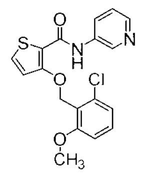
1-63

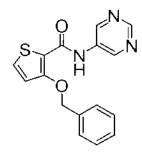
1-64

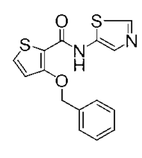
1-65

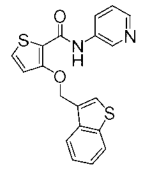
1-66

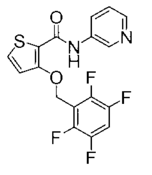
1-67

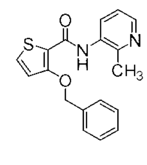
1-68

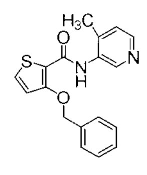
1-69

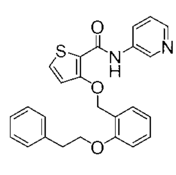
1-70

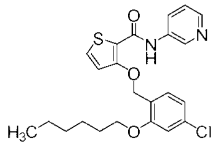

1-71

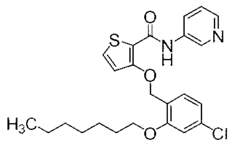

1-72

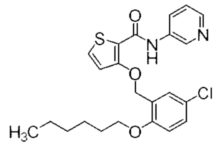

1-73

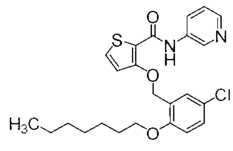

1-74

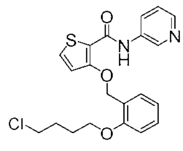

1-75

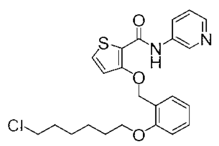

1-76

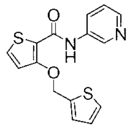

1-77

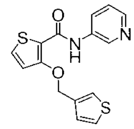

1-78

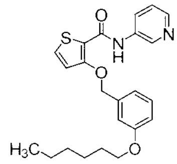

1-79

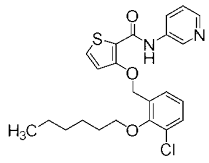

1-80

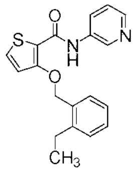

1-81

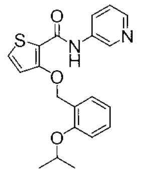

1-82

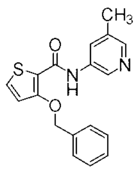

1-83

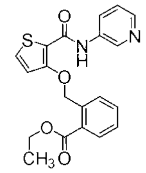

1-84

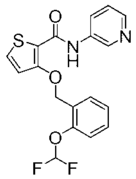

1-85

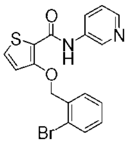

1-86

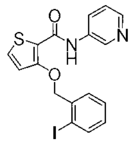

1-87

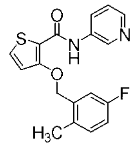

1-88

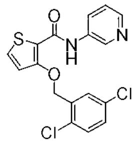

1-89

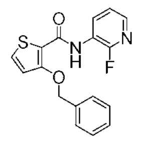

1-90

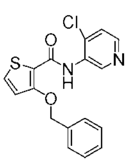

1-91

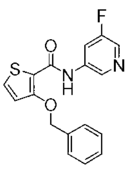

1-92

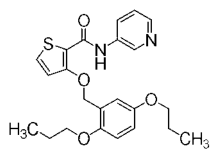

1-93

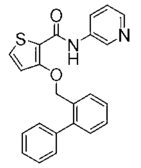

1-94

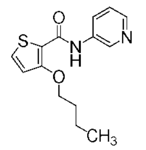

1-95

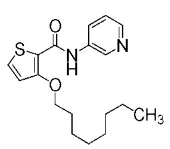

1-96

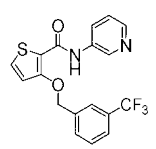

1-97

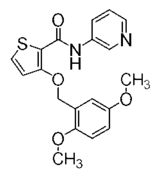

1-98

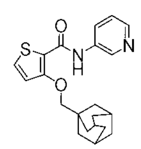

1-99

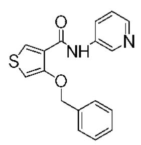

1-100

.

6. The alkoxy thiophene acyl arylamine compound or a pharmaceutically acceptable salt thereof according to claim 1, **characterized in that** the pharmaceutically acceptable salt is a hydrochloride salt, a hydrobromide salt, a tartrate salt, a mesylate salt, a sodium salt, a potassium salt, a calcium salt, an ammonium salt, or an amino acid salt.

7. A pharmaceutical composition comprising a therapeutically effective amount of at least one of the alkoxy thiophene acyl arylamine compound of Formula (I) or a pharmaceutically acceptable salt thereof according to any one of claims 1

to 6.

8. The alkoxy thiophene acyl arylamine compound or a pharmaceutically acceptable salt thereof according to any one of claims 1 to 6 for use in the preparation of a small-molecule inhibitor of sphingomyelin synthase.

9. The alkoxy thiophene acyl arylamine compound or a pharmaceutically acceptable salt thereof according to any one of claims 1 to 6 for use in the preparation of medicaments for preventing or treating diseases caused by abnormal increase in sphingomyelin levels.

10. The use of the alkoxy thiophene acyl arylamine compound or a pharmaceutically acceptable salt thereof according to claim 9, **characterized in that** the diseases caused by abnormal increase in sphingomyelin levels include atherosclerosis, obesity, insulin resistance, fatty liver disease, type II diabetes, enteritis, pulmonary edema, lung injury, malignant tumor, alzheimer’s disease, and dry eye syndrome.

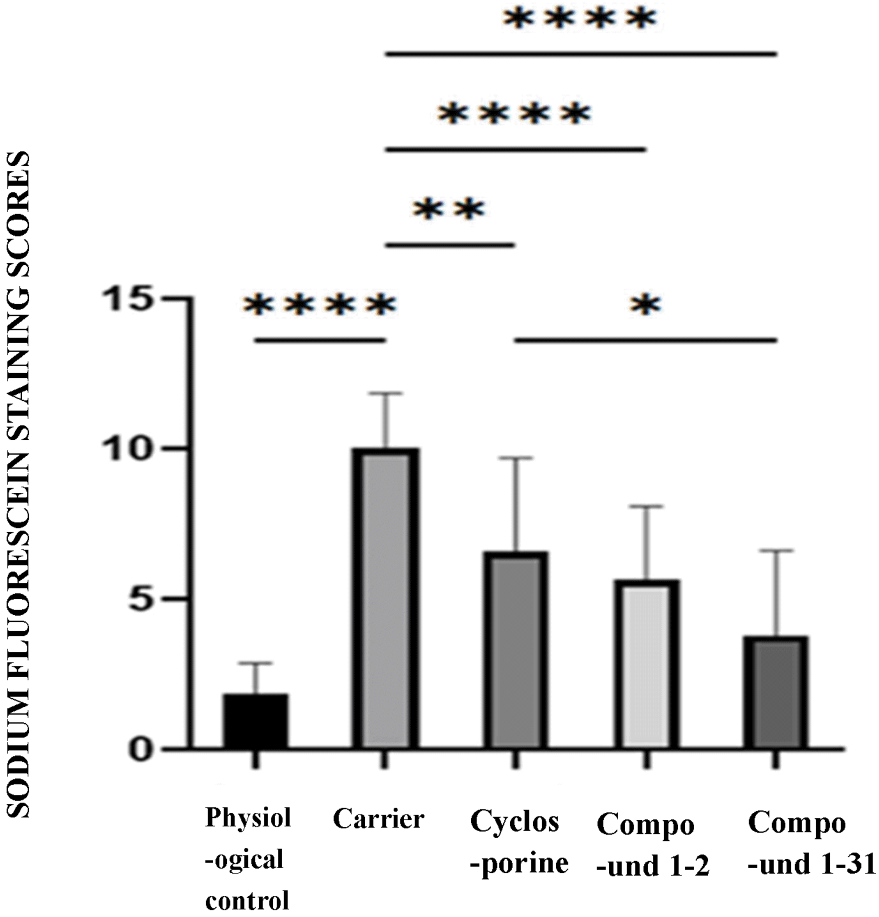
SODIUM FLUORESCEIN STAINING SCORES
****
****
**
****
*
15
10
5
0
Physiol
-ogical
control
Carrier
Cyclos
-porine
Compo
-und 1-2
Compo
-und 1-31

FIG. 1

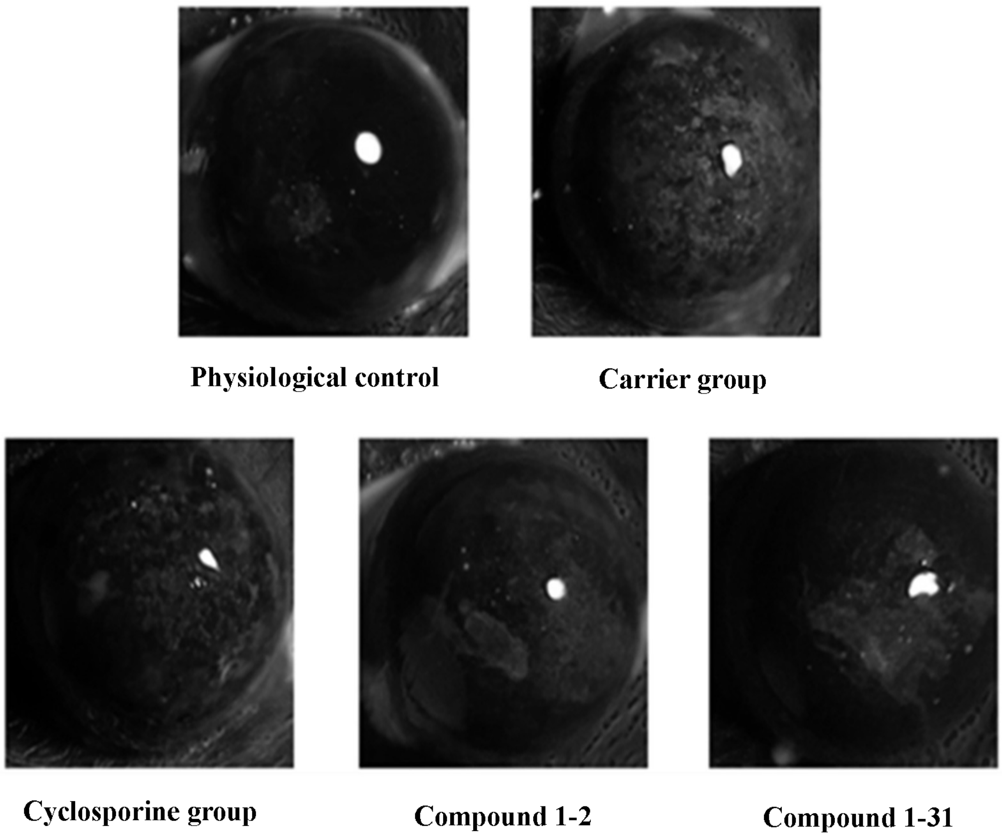
Physiological control
Carrier group
Cyclosporine group
Compound 1-2
Compound 1-31

FIG. 2

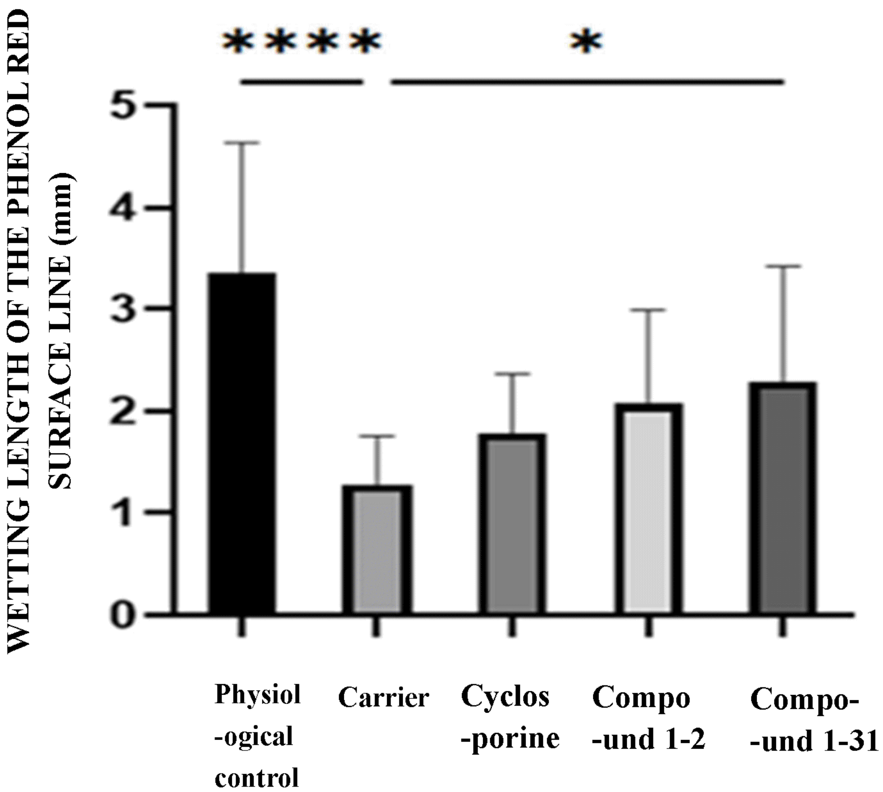
WETTING LENGTH OF THE PHENOL RED SURFACE LINE (mm)
****
*
5
4
3
2
1
0
Physiol -ogical control
Carrier
Cyclos -porine
Compo -und 1-2
Compo- -und 1-31

FIG. 3

**INTERNATIONAL SEARCH REPORT**

International application No. **PCT/CN2024/106985**

**A. CLASSIFICATION OF SUBJECT MATTER**

C07D 409/12(2006.01)i; A61K 31/4406(2006.01)i; A61K 31/5377(2006.01)i; A61K 31/505(2006.01)i; A61P 9/10(2006.01)i; A61P 1/16(2006.01)i; A61P 3/04(2006.01)i; A61P 3/10(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNTXT; VEN; WOTXT; USTXT; EPTXT; CNKI; 万方, WANFANG; STN: 复旦大学, 周璐, 洪佳旭, 杨金童, 王威, 张昕晨, 叶德泳, 噻吩, 甲酰, 苯胺, 烷氧, 鞘磷脂合酶, SMS, 鞘磷脂, 动脉粥样硬化, AS, Thiophene+, Formyl+, Aniline+, Alkoxy+, Sphingomyelin synthase, atherosclerosis, 结构式, structural formula

**C. DOCUMENTS CONSIDERED TO BE RELEVANT**

| Category* | Citation of document, with indication, where appropriate, of the relevant passages | Relevant to claim No. |
|---|---|---|
| X | CN 109906216 A (COLUMBIA UNIVERSITY SENATE) 18 June 2019 (2019-06-18) claims 1-3 and 8-55 | 1-10 |
| X | ACS. "RN2816018-79-6, RN2805842-53-7 et al." *REGISTRY*, 31 August 2022 (2022-08-31), pages 1-23 | 1-4 |
| X | WO 2012034961 A1 (BASF SE et al.) 22 March 2012 (2012-03-22) description, page 108, tableI Ex49 | 1-3 |
| X | US 4144235 A (AMERICAN CYANAMID CO.) 13 March 1979 (1979-03-13) description, columns 8, 9, 11, 12, 15, 17 and 18, embodiments 1, 2, 4, 7, 9, 19, 20, 23 and 24 | 1-4 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 October 2024** | **23 October 2024** |

| Name and mailing address of the ISA/CN | Authorized officer |
|---|---|
| **China National Intellectual Property Administration (ISA/CN)<br>China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/106985**

**C. DOCUMENTS CONSIDERED TO BE RELEVANT**

| Category* | Citation of document, with indication, where appropriate, of the relevant passages | Relevant to claim No. |
|---|---|---|
| X | PRESS, J. B. et al. "Thiophene Systems.4.Selective Syntheses of Thieno[3,4-b][1,4]- and [1,5]Benzodiazepinones(1)" *Journal of Heterocyclic Chemistry,* Vol. 17,, No. 7, 30 November 1980 (1980-11-30), 1361-1364 ISSN: 0022-152X, page 1361, SHEEME II | 1-3, |
| X | PRESS, J. B. et al. "Thiophene Systems.6.An Unexpected Bromine Migration During the Synthesis of Thieno[3,4-b][1,5]Benzoxazepin-10-Ones (1)" *Journal of Heterocyclic Chemistry,* Vol. 18, No. 6, 31 October 1981 (1981-10-31), 1261-1262 ISSN: 0022-152X, page 1261 | 1-3 |
| X | US 4219656 A (AMERICAN CYANAMID CO.) 26 August 1980 (1980-08-26) claim 2, and abstract | 1-3, 7 |
| A | CN 105348182 A (FUDAN UNIVERSITY) 24 February 2016 (2016-02-24) entire document | 1-10 |
| A | CN 110117278 A (FUDAN UNIVERSITY) 13 August 2019 (2019-08-13) entire document | 1-10 |
| A | CN 108329330 A (FUDAN UNIVERSITY) 27 July 2018 (2018-07-27) entire document | 1-10 |
| A | CN 111481548 A (FUDAN UNIVERSITY) 04 August 2020 (2020-08-04) entire document | 1-10 |
| A | CN 113616652 A (SHIJIAZHUANG YILING PHARMACEUTICAL CO., LTD.) 09 November 2021 (2021-11-09) entire document | 1-10 |

**INTERNATIONAL SEARCH REPORT**
**Information on patent family members**

International application No.
**PCT/CN2024/106985**

| Patent document cited in search report | | | Publication date (day/month/year) | Patent family member(s) | | | Publication date (day/month/year) |
|---|---|---|---|---|---|---|---|
| CN | 109906216 | A | 18 June 2019 | WO | 2018017858 | A1 | 25 January 2018 |
| | | | | JP | 2022120072 | A | 17 August 2022 |
| | | | | EP | 3487836 | A1 | 29 May 2019 |
| | | | | EP | 3487836 | A4 | 22 July 2020 |
| | | | | AU | 2017298473 | A1 | 24 January 2019 |
| | | | | IL | 292457 | A | 01 June 2022 |
| | | | | IL | 264310 | B | 01 June 2022 |
| | | | | CA | 3031365 | A1 | 25 January 2018 |
| | | | | US | 2018021273 | A1 | 25 January 2018 |
| | | | | US | 10653648 | B2 | 19 May 2020 |
| | | | | KR | 20190030693 | A | 22 March 2019 |
| | | | | JP | 2019527226 | A | 26 September 2019 |
| | | | | IL | 264310 | A | 31 March 2019 |
| | | | | HK | 40008477 | A0 | 12 June 2020 |
| | | | | MX | 2019000864 | A1 | 03 June 2019 |
| WO | 2012034961 | A1 | 22 March 2012 | US | 2013178502 | A1 | 11 July 2013 |
| | | | | EP | 2616459 | A1 | 24 July 2013 |
| | | | | JP | 2013542918 | A | 28 November 2013 |
| | | | | US | 8609700 | B2 | 17 December 2013 |
| | | | | EP | 2616459 | B1 | 04 May 2016 |
| | | | | IN | 201301384 | P4 | 03 June 2016 |
| | | | | BR | 112013005382 | A2 | 07 June 2016 |
| | | | | BR | 112013005382 | B1 | 08 February 2020 |
| US | 4144235 | A | 13 March 1979 | | None | | |
| US | 4219656 | A | 26 August 1980 | | None | | |
| CN | 105348182 | A | 24 February 2016 | WO | 2016029767 | A1 | 03 March 2016 |
| | | | | CA | 2958927 | A1 | 03 March 2016 |
| | | | | CA | 2958927 | C | 05 September 2023 |
| | | | | JP | 2017529333 | A | 05 October 2017 |
| | | | | US | 2017253564 | A1 | 07 September 2017 |
| | | | | US | 10196359 | B2 | 05 February 2019 |
| | | | | EP | 3196191 | A1 | 26 July 2017 |
| | | | | EP | 3196191 | A4 | 25 April 2018 |
| | | | | EP | 3196191 | B1 | 19 April 2023 |
| | | | | KR | 20170042631 | A | 19 April 2017 |
| | | | | KR | 102544838 | B1 | 16 June 2023 |
| | | | | CN | 105348182 | B | 13 April 2018 |
| | | | | JP | 6644770 | B2 | 12 February 2020 |
| CN | 110117278 | A | 13 August 2019 | JP | 2021512913 | A | 20 May 2021 |
| | | | | JP | 7475049 | B2 | 26 April 2024 |
| | | | | EP | 3750890 | A1 | 16 December 2020 |
| | | | | EP | 3750890 | A4 | 11 August 2021 |
| | | | | US | 2021032230 | A1 | 04 February 2021 |
| | | | | US | 11396504 | B2 | 26 July 2022 |
| | | | | WO | 2019154047 | A1 | 15 August 2019 |
| | | | | CN | 110117278 | B | 19 July 2022 |
| CN | 108329330 | A | 27 July 2018 | CN | 108329330 | B | 04 May 2021 |
| CN | 111481548 | A | 04 August 2020 | | None | | |
| CN | 113616652 | A | 09 November 2021 | WO | 2021223713 | A1 | 11 November 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021165292 A
- WO 2005032492 A

### Non-patent literature cited in the description

- **XIAODONG DENG et al.** *Analytical Letters*, 2012, vol. 45 (12), 1581-1589